# EUROPEAN PATENT SPECIFICATION

(11) **EP 3 266 419 B1**
(45) Date of publication and mention of the grant of the patent: **09.09.2020**
(21) Application number: 17167442.7
(22) Date of filing: 25.02.2010
(51) Int. Cl.: A61F 2/30

(54) **PATIENT-ADAPTED AND IMPROVED ORTHOPEDIC IMPLANTS**
PATIENTENADAPTIERTE UND VERBESSERTE ORTHOPÄDISCHE IMPLANTATE
IMPLANTS ORTHOPÉDIQUES AMÉLIORÉS ET ADAPTÉS AU PATIENT

(30) Priority: 25.02.2009 US 155362 P; 24.06.2009 US 269405 P; 31.07.2009 US 273216 P; 26.08.2009 US 275174 P; 04.11.2009 US 280493 P; 18.12.2009 US 284458 P
(43) Date of publication of application: 10.01.2018
(62) Divisional of application: 10746859.7
(73) Proprietor: ConforMIS, Inc., Billerica, MA 01821 (US)
(72) Inventor: BOJARSKI, Raymond, Attleboro, MA 02703 (US); LANG, Philipp, Lexington, MA 02421 (US); HAO, Nam, Marlborough, MA 01752 (US); FITZ, Wolfgang, Sherborn, MA 01770 (US); SLAMIN, John, Wretham, MA 02093 (US); STEINES, Daniel, Lexington, MA 02421 (US)
(74) Representative: Grund, Martin

(56) References cited:
- WO-A2-01/77988
- US-A- 4 822 365
- US-A1- 2005 197 814

## Description

### TECHNICAL FIELD

The invention relates to improved and/or patient-adapted (e.g., patient-specific and/or patient-engineered) orthopedic implants as well as related methods, designs and models.

### BACKGROUND

Generally, a diseased, injured or defective joint, such as, for example, a joint exhibiting osteoarthritis, has been repaired using standard off-the-shelf implants and other surgical devices. Specific off-the-shelf implant designs have been altered over the years to address particular issues. However, in altering a design to address a particular issue, historical design changes frequently have created one or more additional issues for future designs to address. Collectively, many of these issues have arisen from one or more differences between a patient's existing or healthy joint anatomy and the corresponding features of an implant component.

US 2005/01797814 discloses a system which improves the design of artificial implant components for use in joint replacement surgeries. Said system includes an anthropometric static image data analyzer, an implant model generator, a kinematic model simulator, and a dynamic response data analyzer.

### SUMMARY

The invention is defined by the appended claims. The patient-adapted (e.g., patient-specific and/or patient-engineered) implant components described herein can be selected (e.g., from a library), designed (e.g., preoperatively designed including, optionally, manufacturing the components or tools), and/or selected and designed (e.g., by selecting a blank component or tool having certain blank features and then altering the blank features to be patient-adapted).

Patient-adapted features of an implant component, or related method can be achieved by analyzing imaging test data and selecting and/or designing (e.g., preoperatively selecting from a library and/or designing) an implant component, having a feature that is matched and/or optimized for the particular patient's biology. The imaging test data can include data from the patient's joint, for example, data generated from an image of the joint such as x-ray imaging, cone beam CT, digital tomosynthesis, and ultrasound, a MRI or CT scan or a PET or SPECT scan, is processed to generate a varied or corrected version of the joint or of portions of the joint or of surfaces within the joint. Disclosed is a method to create a desired model of a joint or of portions or surfaces of a joint based on data derived from the existing joint. For example, the data can also be used to create a model that can be used to analyze the patient's joint and to devise and evaluate a course of corrective action. The data and/or model also can be used to design an implant component having one or more patient-specific features, such as a surface or curvature.

The articular implant component can be a knee joint implant component, a hip joint implant component, a shoulder joint implant component, or a spinal implant component. For example, the pre-primary articular implant component can be a knee joint implant component, such as a femoral implant component.

It is to be understood that the features of the various aspects described herein are not mutually exclusive and may exist in various combinations and permutations.

### BRIEF DESCRIPTION OF THE DRAWINGS

The foregoing and other objects, aspects, features, and advantages of aspects will become more apparent and may be better understood by referring to the following description, taken in conjunction with the accompanying drawings, in which:
**FIG. 1** is a flow chart illustrating a process that includes selecting and/or designing an initial patient-adapted implant, in accordance with an aspect of the invention;
**FIGS. 2A-2C** schematically represent three illustrative aspects of implants and/or implant components, in accordance with various aspects of the invention;
**FIGS. 3A- 3B** depict designs of implant components that have six bone cuts **(****FIG. 3A****),** seven bone cuts **(****FIG. 3B****),** in accordance with various aspects of the invention;

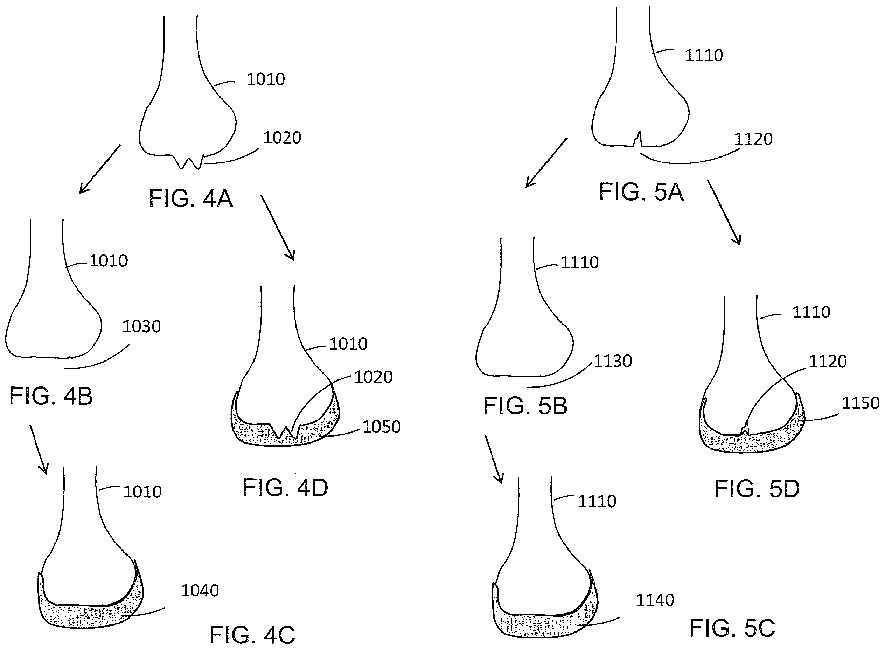

**FIG. 4A** is a drawing of a cross-sectional view of an end of a femur with an osteophyte; **FIG. 4B** is a drawing of the end of the femur of **FIG. 4A** with the osteophyte virtually removed; **FIG. 4C** is a drawing of the end of the femur of **FIG. 4B** with the osteophyte virtually removed and showing a cross-sectional view of an implant designed to the shape of the femur with the osteophyte removed; **FIG. 4D** is a drawing of the end of the femur of **FIG. 4A** and shows a cross-sectional view of an implant designed to the shape of the femur with the osteophyte intact, in accordance with various aspects of the invention;
**FIG. 5A** is a drawing of a cross-sectional view of an end of a femur with a subchondral void in the bone; **FIG. 5B** is a drawing of the end of the femur of **FIG. 5A** with the void virtually removed; **FIG. 5C** is a drawing of the end of the femur of **FIG. 5B** with the void virtually removed and showing a cross-sectional view of an implant designed to the shape of the femur with the void removed; **FIG. 5D** is a drawing of the end of the femur of **FIG. 5A** and showing a cross-sectional view of an implant designed to the shape of the femur with the void intact, in accordance with various aspects of the invention;
**FIG. 6** illustrates a coronal plane of the knee with exemplary resection cuts that can be used to correct lower limb alignment in a knee replacement;
**FIG. 7** depicts a coronal plane of the knee shown with femoral implant medial and lateral condyles having different thicknesses to help to correct limb alignment;

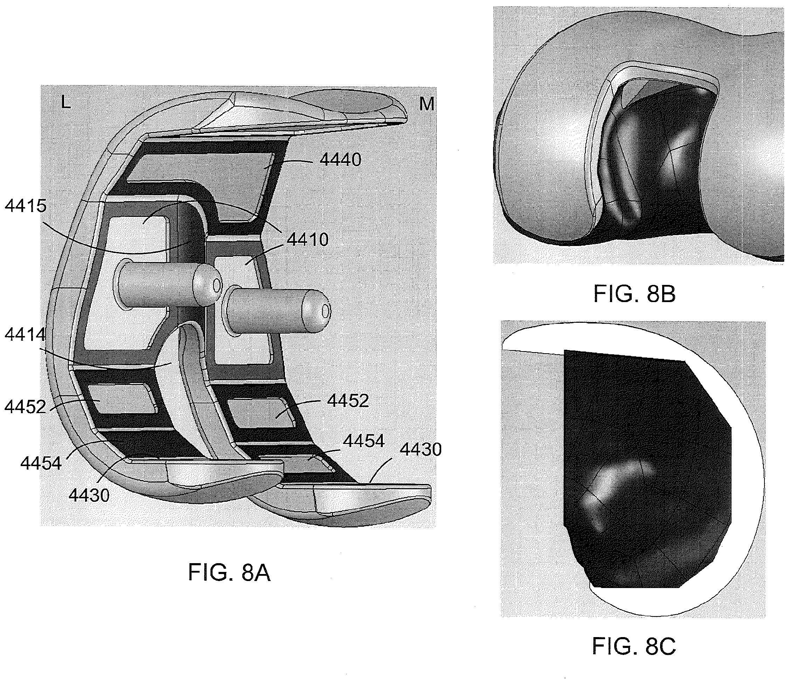

**FIGS. 8A-8C** illustrate a femoral implant component having six bones cuts that include one or more parallel and non-coplanar facets;

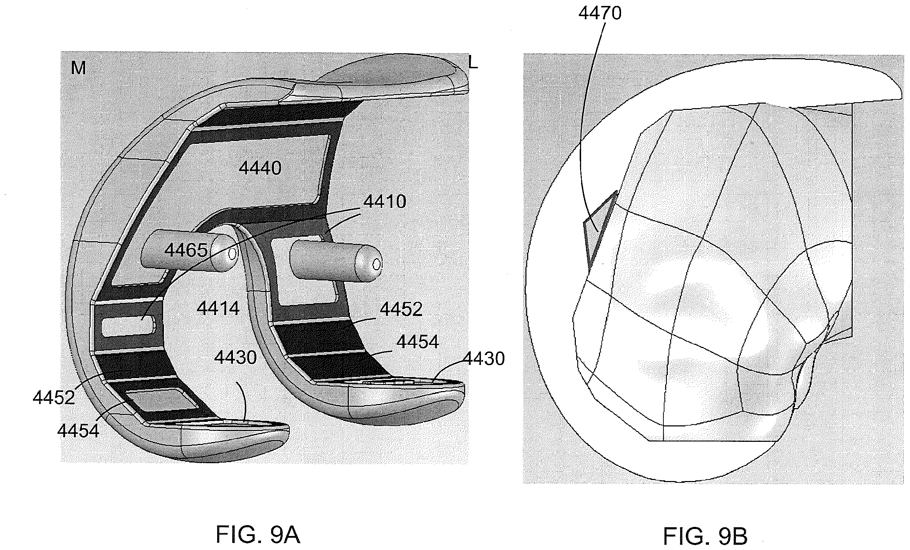

**FIGS. 9A-9B** illustrate a femoral implant component having seven bones cuts that include one or more parallel and non-coplanar facets;

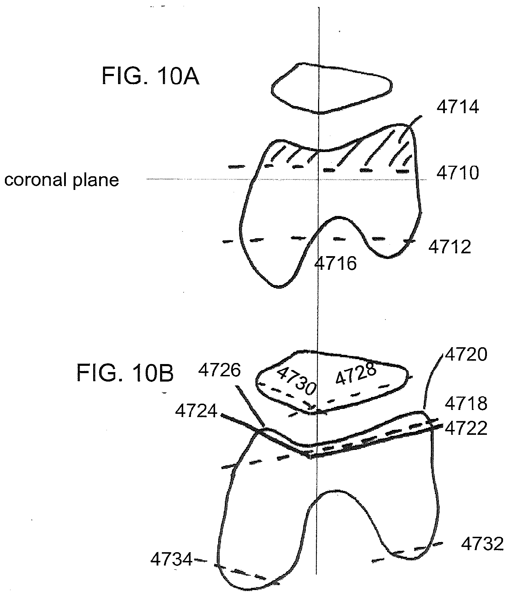

**FIGS. 10A** and **10B** are schematic views of a femur and patella and exemplary resection cut planes;

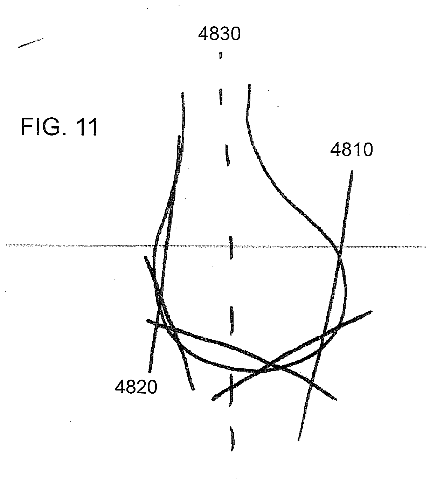

**FIG. 11** is a schematic view of a sagittal plane of a femur with facet cuts indicated;
**FIG. 12** is a flow chart illustrating an exemplary process for selecting and/or designing a patient-adapted total knee implant, in accordance with an aspect of the invention;
**FIG. 13A** illustrates a distal femur and a distal resection plane parallel to the epicondylar axis; **FIG. 13B** shows an example of an anterior oblique resection plane **8830.**
**FIGS. 14A** to **14E** show optimized resection cut planes to a patient's femur based on the medial condyle.
**FIGS. 15A** and 15**B** show resection cut planes for a patient's lateral condyle posterior chamfer **(****FIG. 15A****)** and lateral condyle posterior **(****FIG. 15B****)** cut planes that are independently optimized based on patient-specific data for the lateral condyle;
**FIGS. 16A** and **16B** illustrate two exemplary distal resection cut planes for two different cut designs;
**FIGS. 17A** and 17**B** illustrate five femoral resection cuts for the two designs shown in **FIGS. 16A** and **16B****,** respectively;
**FIGS. 18A** and **18B** illustrate the completed cut femur models for each of two cut designs;
**FIG. 19A** illustrates an aspect of a cut plane design having anterior and posterior cut planes that diverge from the component peg axis; **FIG. 19B** illustrates an implant component design that includes a peg diameter of 7 mm with a rounded tip, in accordance with an aspect of the invention;
**FIGS. 20A** and **20B** illustrate exemplary bone-facing surfaces of femoral implant component designs that include a patient-adapted peripheral margin, in accordance with various aspects of the invention;
**FIGS. 21A** and **21B** illustrate side views of exemplary femoral implant component designs, in accordance with an aspect of the invention;
**FIG. 22** illustrates a femoral implant component (PCL-retaining) having seven bone cuts, in accordance with various aspects of the invention;
**FIG. 23A** and **FIG. 23B** illustrate the resection cut planes for the implant component of **FIG. 22****;**
**FIG. 24** illustrates the implant component of **FIG. 22** from a different angle to show cement pocket and peg features, in accordance with an aspects of the invention;
**FIG. 25A** shows a five-cut-plane femoral resection design for a femoral implant component having five bone cuts; **FIG. 25B** shows a seven-cut-plane femoral resection design for a femoral implant component having seven bone cuts;
**FIG. 26A** shows a patient's femur having five, not flexed resection cuts; **FIG. 26B** shows the same femur but with five, flexed resection cuts;

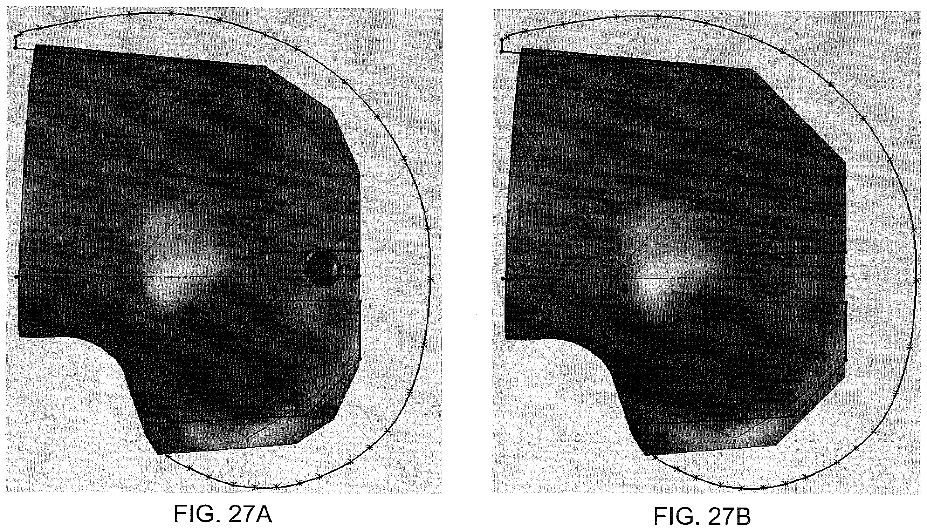

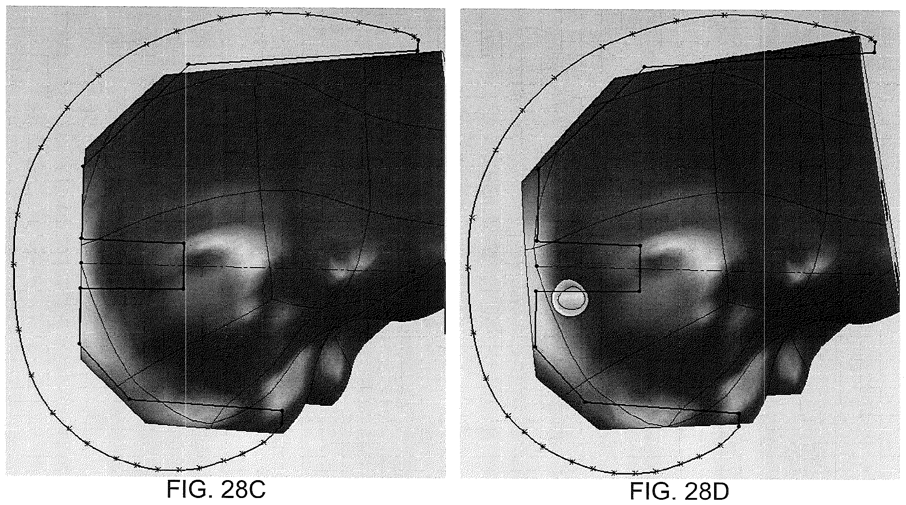

**FIGS. 27A** to **27D** show outlines of a traditional five-cut femoral component (in hatched lines) overlaid with, in **27A,** a femur having seven optimized resection cuts for matching an optimized seven-bone-cut implant component; in **FIG. 27B****,** a femur having five optimized resection cuts for matching to an optimized five-bone-cut implant component; in **FIG. 27C,** a femur having five, not flexed resection cuts for matching to an optimized five-bone-cut implant component; and in **FIG. 27D,** a femur having five, flexed resection cuts for matching to an optimized five-bone-cut, flexed implant component, in accordance with various aspects of the invention;
Additional figure descriptions are included in the text below. Unless otherwise denoted in the description for each figure, "M" and "L" in certain figures indicate medial and lateral sides of the view; "A" and "P" in certain figures indicate anterior and posterior sides of the view, and "S" and "I" in certain figures indicate superior and inferior sides of the view.

### DETAILED DESCRIPTION

### The invention is defined by the appended claims.

### Introduction

When a surgeon uses a traditional off-the-shelf implant to replace a patient's joint, for example, a knee joint, hip joint, or shoulder joint, certain features of the implant typically do not match the particular patient's biological features. These mismatches can cause various complications during and after surgery. For example, surgeons may need to extend the surgery time and apply estimates and rules of thumb during surgery to address the mismatches. For the patient, complications associated with these mismatches can include pain, discomfort, soft tissue impingement, and an unnatural feeling of the joint during motion, e.g., so-called mid-flexion instability, as well as an altered range of movement and an increased likelihood of implant failure. In order to fit a traditional implant component to a patient's articular bone, surgeons typically remove substantially more of the patient's bone than is necessary to merely clear diseased bone from the site. This removal of substantial portions of the patient's bone frequently diminishes the patient's bone stock to the point that only one subsequent revision implant is possible.

Disclosed are related methods of designing (e.g., designing and making)the implants disclosed herein that can be applied to any joint including, without limitation, a spine, spinal articulations, an intervertebral disk, a facet joint, a shoulder, an elbow, a wrist, a hand, a finger, a hip, a knee, an ankle, a foot, or a toe joint. Disclosed are methods, for resectioning the patient's anatomy in order to implant the implant components disclosed herein.

The implant components and/or related methods described herein can include a combination of patient-specific and patient-engineered features. For example, patient-specific data collected preoperatively can be used to engineer one or more optimized surgical cuts to the patient's bone and to design or select a corresponding implant component having or more bone-facing surfaces or facets (i.e., "bone cuts") that specifically match one or more of the patient's resected bone surfaces. The surgical cuts to the patient's bone can be optimized (i.e., patient-engineered) to enhance one or more parameters, such as: (1) deformity correction and limb alignment (2) maximizing preservation of bone, cartilage, or ligaments, or (3) restoration and/or optimization of joint kinematics or biomechanics.

### Improved implants and related methods

Disclosed are implants, and related methods that can be used to provide to a patient a pre-primary procedure and/or a pre-primary implant such that a subsequent, replacement implant can be performed with a second (and, optionally, a third, and optionally, a fourth) patient-adapted pre-primary implant or with a traditional primary implant. Disclosed is a first pre-primary joint-replacement procedure that includes a or related method. The related method can be preoperatively designed from patient-specific data, such as one or more images of the patient's joint, to include one or more features that are patient-specific or patient-engineered. The features (e.g., dimensions, shape, surface contours) of the first pre-primary implant andpatient-specific data (e.g., features of the patient's resected bone surfaces and features of the patient's contralateral joint) can be stored in a database. When the first pre-primary implant fails, for example, due to bone loss or osteolysis or aseptic loosening at a later point in time (e.g., 15 years after the original implantation) a second implant can be implanted. For the second implant procedure, the amount of diseased bone can be assessed. If the amount of diseased bone to be resected is minimal, the patient-specific data can be used to select and/or design a second pre-primary procedure and/or a pre-primary implant. If the amount of diseased bone to be resected is substantial, a traditional primary procedure and a traditional implant can be employed.

Implants and related methods that can be used to provide to a patient a primary procedure and/or a primary implant such that a subsequent replacement implant can be used as part of a traditional revision procedure. Disclosed are implants related methods that can be used to provide a patient-adapted revision implant. For example, following a traditional implant, a subsequent revision can include a patient-adapted procedure and/or a patient-adapted implant component as described herein.

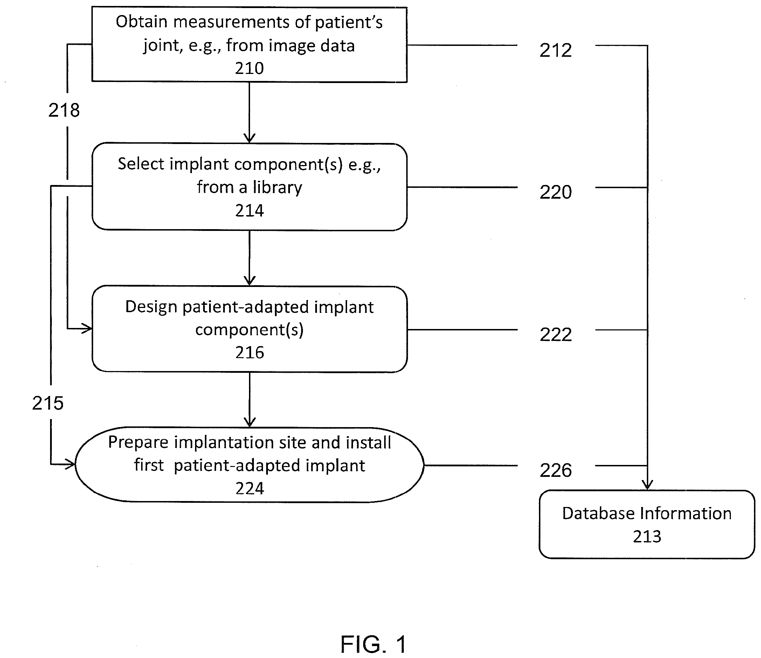

**FIG. 1** is a flow chart illustrating a process that includes selecting and/or designing a first patient-adapted implant, for example, a pre-primary implant. First, using the techniques described herein or those suitable and known in the art, measurements of the target joint are obtained **210.** This step can be repeated multiple times, as desired. Optionally, a virtual model of the joint can be generated, for example, to determine proper joint alignment and the corresponding resection cuts and implant component features based on the determined proper alignment. This information can be collected and stored **212** in a database **213.** Once measurements of the target joint are obtained and analyzed to determine resection cuts and patient-adapted implant features, the patient-adapted implant components can be selected **214** (e.g., selected from a virtual library and optionally manufactured without further design alteration **215,** or selected from a physical library of implant components). Alternatively, or in addition, one or more implant components with best-fitting and/or optimized features can be selected **214** (e.g., from a library) and then further designed (e.g., designed and manufactured) **216.** Alternatively or in addition, one or more implant components with best-fitting and/or optimized features can be designed (e.g., designed and manufactured) **218, 216** without an initial selection from a library. Using a virtual model to assess the designed implant component(s), this process also can be repeated as desired (e.g., before one or more physical components are selected and/or generated). The information regarding the designed implant component(s) can be collected and stored **220, 222** in a database **213.** Once a desired first patient-adapted implant component or set of implant components is obtained, a surgeon can prepare the implantation site and install the first implant **224.** The information regarding preparation of the implantation site and implant installation can be collected and stored **226** in a database **213.** In this way, the information associated with the first pre-primary implant component is available for use by a surgeon for subsequent implantation of a second pre-primary or a primary implant.

The term "implant component" as used herein can include: (i) one of two or more devices that work together in an implant or implant system, or (ii) a complete implant or implant system, for example, in aspects in which an implant is a single, unitary device. The term "match" as used herein is envisioned to include one or both of a negative-match, as a convex surface fits a concave surface, and a positive-match, as one surface is identical to another surface.

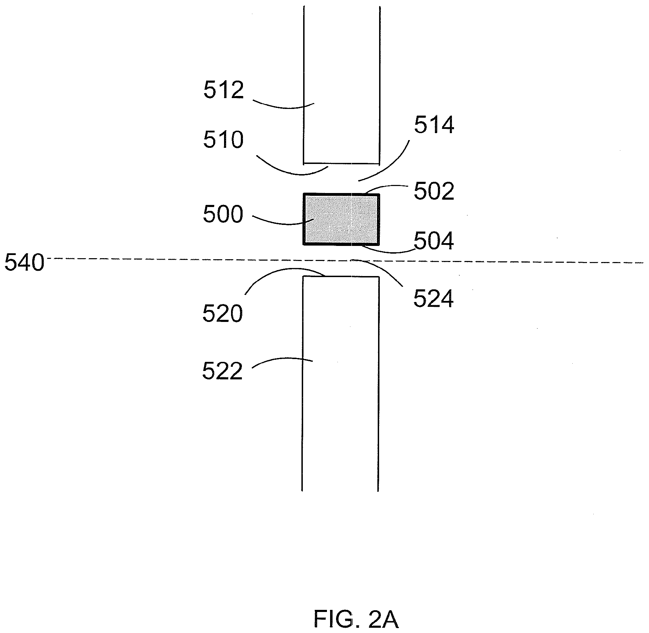

Three implants and/or implant components are schematically represented in **FIGS. 2A-2C****.** In **FIG. 2A****,** the illustrative implant component **500** includes an inner, bone-facing surface **502** and an outer, joint-facing surface **504.** The inner bone-facing surface **502** engages a first articular surface **510** of a first biological structure **512,** such as bone or cartilage, at a first interface **514.** The articular surface **510** can be a native surface, a resected surface, or a combination of the two. The outer, joint-facing surface **504** opposes a second articular surface **520** of a second biological structure **522** at a joint interface **524.** The dashed line across each figure illustrates a patient's joint-line. Disclosed is that one or more features of the implant component, for example, an M-L, A-P, or S-I dimension, a feature of the inner, bone-facing surface **502,** and/or a feature of the outer, joint-facing surface **504,** are patient-adapted (i.e., include one or more patient-specific and/or patient-engineered features).

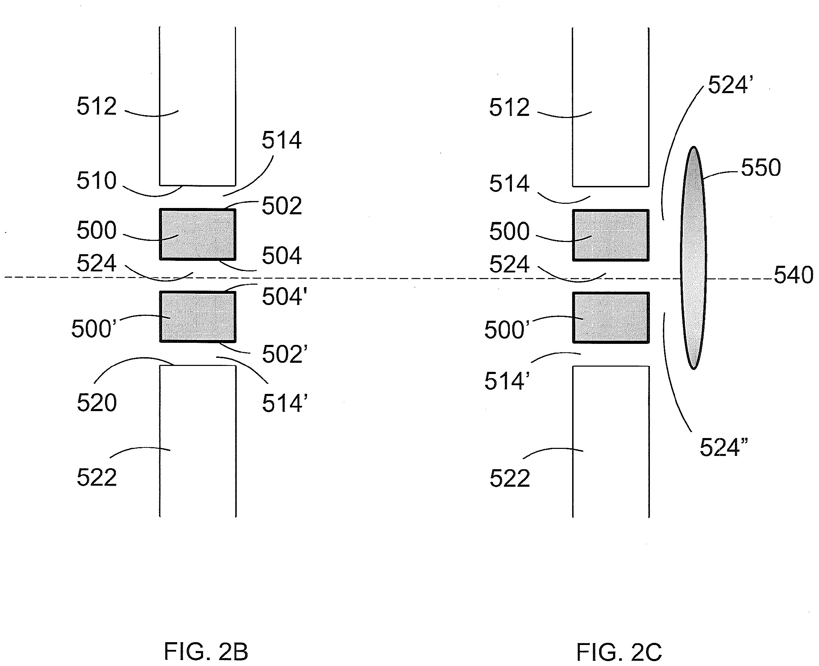

**FIG. 2B** shows two implant components **500, 500'.** Each implant component **500, 500'** includes an inner, bone-facing surface **502, 502'** and an outer, joint-facing surface **504, 504'.** The first inner, bone-facing surface **502** engages a first articular surface **510** of a first biological structure **512** (e.g., bone or cartilage) at a first interface **514.** The first articular surface **510** can be a native surface, a cut surface, or a combination of the two. The second bone-facing surface **502'** engages a second articular surface **520** of a second biological structure **522** at a second interface **514'.** The second articular surface **520** can be a native surface, a resected surface, or a combination of the two. In addition, an outer, joint-facing surface **504** on the first component **500** opposes a second, outer joint-facing surface **504'** on the second component **500'** at the joint interface **524.** Disclosed is that one or more features of the implant component, for example, one or both of the inner, bone-facing surfaces **502, 502'** and/or one or both of the outer, joint-facing surfaces **504, 504',** are patient-adapted (i.e., include one or more patient-specific and/or patient-engineered features).

**FIG. 2C** includes the two implant components **500, 500',** the two biological structures **512, 522,** the two interfaces **514, 514',** and the joint interface **524,** as well as the corresponding surfaces, as described in **FIG. 2B****.** However, **FIG. 2C** also includes structure **550,** which can be an implant component or a biological. Accordingly, the presence of a third structural **550** surface in the joint creates a second joint interface **524',** and possibly a third **524",** in addition to joint interface **524.** Alternatively or in addition to the patient-adapted features described above for components **500** and **500',** the components **500, 500'** can include one or more features, such as surface features at the additional joint interface(s) **524, 524",** as well as other dimensions (e.g., height, width, depth, contours, and other dimensions) that are patient-adapted, in whole or in part. Moreover, structure **550,** when it is an implant component, also can have one or more patient-adapted features, such as one or more patient-adapted surfaces and dimensions.

Traditional implants and implant components can have surfaces and dimensions that are a poor match to a particular patient's biological feature(s). The patient-adapted implants, related methods disclosed herein improve upon these deficiencies. The following two subsections describe two particular improvements, with respect to the bone-facing surface and the joint-facing surface of an implant component; however, the principles described herein are applicable to any aspect of an implant component.

### Bone-facing surface of an implant component

The bone-facing surface of an implant can be designed to substantially negatively-match one more bone surfaces. For example, at least a portion of the bone-facing surface of a patient-adapted implant component can be designed to substantially negatively-match the shape of subchondral bone, cortical bone, endosteal bone, and/or bone marrow. A portion of the implant also can be designed for resurfacing, for example, by negatively-matching portions of a bone-facing surface of the implant component to the subchondral bone or cartilage. Accordingly, the bone-facing surface of an implant component can include one or more portions designed to engage resurfaced bone, for example, by having a surface that negatively-matches uncut subchondral bone or cartilage, and one or more portions designed to engage cut bone, for example, by having a surface that negatively-matches a cut subchondral bone.

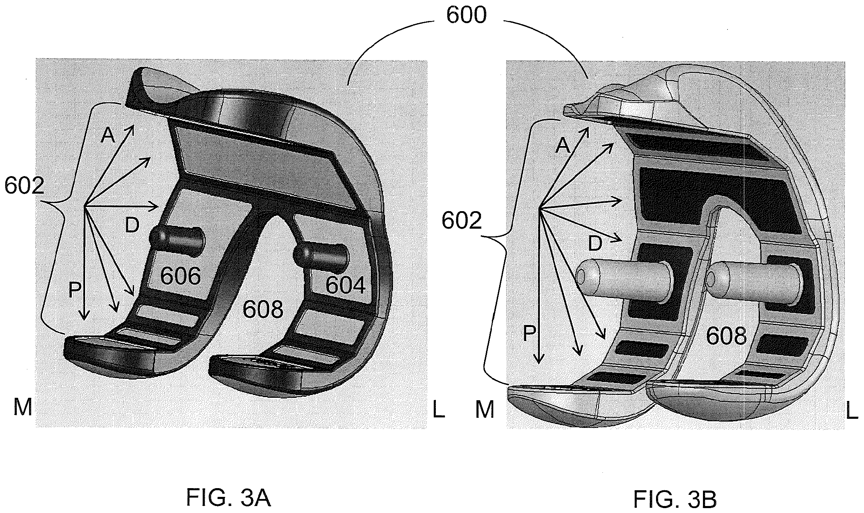

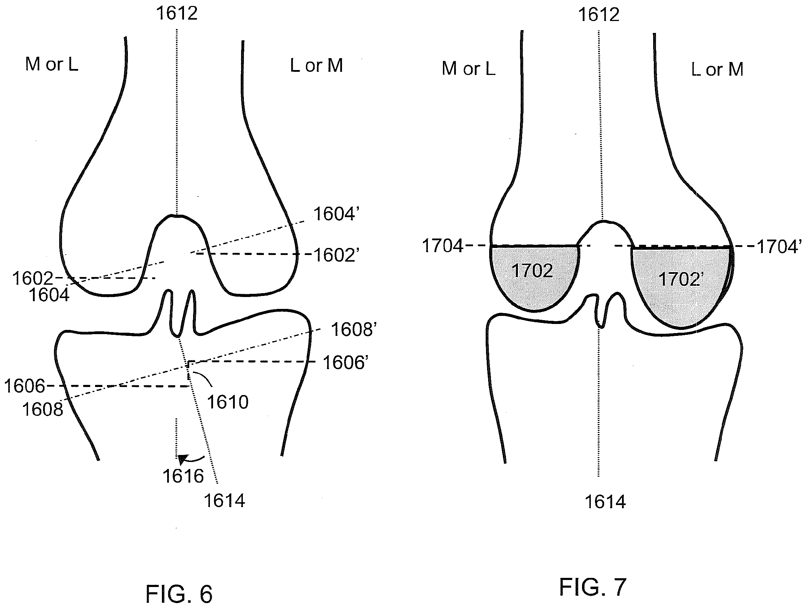

**FIG. 3A** illustrates an exemplary femoral implant component **600** having six bone cuts. **FIG. 3B** illustrates a femoral implant component **600** having seven bone cuts. In **FIG. 3A** and **FIG. 3B****,** the six or seven respective bone cuts are identified by arrows on the inner, bone-facing surface **602** of the implant component **600.** The bone cuts can include, for example, an anterior bone cut **A,** a distal bone cut **D,** and a posterior bone cut **P,** as well as one or more anterior chamfer bone cuts between the anterior bone cut **A** and distal bone cut **D,** and/or one or more posterior chamfer bone cuts between the distal posterior bone cut **P** and the distal bone cut **D.** The implant component depicted in **FIG. 6A** includes one anterior chamfer bone cut and two posterior chamfer bone cuts, in addition to anterior, posterior and distal bone cuts. The implant component depicted in **FIG. 6B** includes two anterior chamfer bone cuts and two posterior chamfer bone cuts, in addition to anterior, posterior and distal bone cuts.

Any one or more bone cuts can include one or more facets. For example, the implant components exemplified in **FIG. 3A** and **FIG. 3B** depict corresponding condylar facets for each of the distal bone cut, posterior bone cut, first posterior chamfer bone cut and second posterior chamfer bone cut. In **FIG. 3A****,** distal bone cut facets on lateral and medial condyles are identified by **604** and **606,** respectively. Facets of a bone cut can be separated by a space between corresponding regions of an implant component, as exemplified by the condylar facets separated by the intercondylar space **608** in **FIG. 3A** and **FIG. 3B****.** Alternatively or in addition, facets of a bone cut can be separated by a step cut, for example, a vertical or angled cut connecting two non-coplanar or non facets of a bone cut. As shown by the implant components exemplified in each of **FIG. 3A** and **FIG. 3B****,** each bone cut and/or bone cut facet can be substantially planar.

Disclosed is that corresponding sections of an implant component can include different thicknesses (i.e., distance between the component's bone-facing surface and joint-facing surface), surface features, bone cut features, section volumes, and/or other features. For example, as shown in **FIG. 3A****,** the corresponding distal lateral and medial sections of the implant, identified by **604** and **606** on their respective bone cut facets, include different thicknesses, section volumes, bone cut angles, and bone cut surface areas. As this example illustrates, one or more of the thicknesses, section volumes, bone cut angles, bone cut surface areas, bone cut curvatures, numbers of bone cuts, peg placements, peg angles, and other features may vary between two or more sections (e.g., corresponding sections on lateral and medial condyles) of an implant component. One, more, or all of these features can be the same in corresponding sections of an implant component. An implant design that allows for independent features on different sections of an implant allows various options for achieving one or more goals, including, for example, (1) deformity correction and limb alignment (2) preserving bone, cartilage, and/or ligaments.

### Joint-facing surface of an implant component

Disclosed is that the joint-facing surface of an implant component can be designed to match the shape of the patient's biological structure. The joint-facing surface can include, for example, the bearing surface portion of the implant component that engages an opposing biological structure or implant component in the joint to facilitate typical movement of the joint. The patient's biological structure can include, for example, cartilage, bone, and/or one or more other biological structures.

For example, the joint-facing surface of an implant component is designed to match the shape of the patient's articular cartilage. For example, the joint-facing surface can substantially positively-match one or more features of the patient's existing cartilage surface and/or healthy cartilage surface and/or a calculated cartilage surface, on the articular surface that the component replaces. Alternatively, it can substantially negatively-match one or more features of the patient's existing cartilage surface and/or healthy cartilage surface and/or a calculated cartilage surface, on the opposing articular surface in the joint. As described below, corrections can be performed to the shape of diseased cartilage by designing surgical steps (and, optionally, patient-adapted surgical tools) to re-establish a normal or near normal cartilage shape that can then be incorporated into the shape of the joint-facing surface of the component. These corrections can be implemented and tested in virtual two-dimensional and three-dimensional models. The corrections and testing can include kinematic analysis and/or surgical steps.

Disclosed is that the joint-facing surface of an implant component can be designed to positively-match the shape of subchondral bone. For example, the joint-facing surface of an implant component can substantially positively-match one or more features of the patient's existing subchondral bone surface and/or healthy subchondral bone surface and/or a calculated subchondral bone surface, on the articular surface that the component attaches to on its bone-facing surface. Alternatively, it can substantially negatively-match one or more features of the patient's existing subchondral bone surface and/or healthy subchondral bone surface and/or a calculated subchondral bone surface, on the opposing articular surface in the joint. Corrections can be performed to the shape of subchondral bone to re-establish a normal or near normal articular shape that can be incorporated into the shape of the component's joint-facing surface. A standard thickness can be added to the joint-facing surface, for example, to reflect an average cartilage thickness. Alternatively, a variable thickness can be applied to the component. The variable thickness can be selected to reflect a patient's actual or healthy cartilage thickness, for example, as measured in the individual patient or selected from a standard reference database.

The joint-facing surface of an implant component can include one or more standard features. The standard shape of the joint-facing surface of the component can reflect, at least in part, the shape of typical healthy subchondral bone or cartilage. For example, the joint-facing surface of an implant component can include a curvature having standard radii or curvature in one or more directions. Alternatively or in addition, an implant component can have a standard thickness or a standard minimum thickness in select areas. Standard thickness(es) can be added to one or more sections of the joint-facing surface of the component or, alternatively, a variable thickness can be applied to the implant component.

Disclosed is in **FIG. 2B** and 2**C** that in addition to a first implant component, a second implant component having an opposing joint-facing surface is included. The second implant component's bone-facing surface and/or joint-facing surface can be designed as described above. Moreover, the joint-facing surface of the second component can be designed, at least in part, to match (e.g., substantially negatively-match) the joint-facing surface of the first component. Designing the joint-facing surface of the second component to complement the joint-facing surface of the first component can help reduce implant wear and optimize kinematics. Thus, the joint-facing surfaces of the first and second implant components can include features that do not match the patient's existing anatomy, but instead negatively-match or nearly negatively-match the joint-facing surface of the opposing implant component.

However, when a first implant component's joint-facing surface includes a feature adapted to a patient's biological feature, a second implant component having a feature designed to match that feature of the first implant component also is adapted to the patient's same biological feature. By way of illustration, when a joint-facing surface of a first component is adapted to a portion of the patient's cartilage shape, the opposing joint-facing surface of the second component designed to match that feature of the first implant component also is adapted to the patient's cartilage shape. When the joint-facing surface of the first component is adapted to a portion of a patient's subchondral bone shape, the opposing joint-facing surface of the second component designed to match that feature of the first implant component also is adapted to the patient's subchondral bone shape. When the joint-facing surface of the first component is adapted to a portion of a patient's cortical bone, the joint-facing surface of the second component designed to match that feature of the first implant component also is adapted to the patient's cortical bone shape. When the joint-facing surface of the first component is adapted to a portion of a patient's endosteal bone shape, the opposing joint-facing surface of the second component designed to match that feature of the first implant component also is adapted to the patient's endosteal bone shape. When the joint-facing surface of the first component is adapted to a portion of a patient's bone marrow shape, the opposing joint-facing surface of the second component designed to match that feature of the first implant component also is adapted to the patient's bone marrow shape.

The opposing joint-facing surface of a second component can substantially negatively-match the joint-facing surface of the first component in one plane or dimension, in two planes or dimensions, in three planes or dimensions, or in several planes or dimensions. For example, the opposing joint-facing surface of the second component can substantially negatively-match the joint-facing surface of the first component in the coronal plane only, in the sagittal plane only, or in both the coronal and sagittal planes.

In creating a substantially negatively-matching contour on an opposing joint-facing surface of a second component, geometric considerations can improve wear between the first and second components. For example, the radii of a concave curvature on the opposing joint-facing surface of the second component can be selected to match or to be slightly larger in one or more dimensions than the radii of a convex curvature on the joint-facing surface of the first component. Similarly, the radii of a convex curvature on the opposing joint-facing surface of the second component can be selected to match or to be slightly smaller in one or more dimensions than the radii of a concave curvature on the joint-facing surface of the first component. In this way, contact surface area can be maximized between articulating convex and concave curvatures on the respective surfaces of first and second implant components.

The bone-facing surface of the second component can be designed to negatively-match, at least in part, the shape of articular cartilage, subchondral bone, cortical bone, endosteal bone or bone marrow (e.g., surface contour, angle, or perimeter shape of a resected or native biological structure). It can have any of the features described above for the bone-facing surface of the first component, such as having one or more patient-adapted bone cuts to match one or more predetermined resection cuts.

Many combinations of first component and second component bone-facing surfaces and joint-facing surfaces are possible. **Table 1** provides illustrative combinations that may be employed.

**Table 1: Illustrative Combinations of Implant Components**

| **1^{st} component bone-facing surface** | **1^{st} component joint-facing surface** | **1^{st} component bone cut(s)** | **2^{nd} component joint facing surface** | **2^{nd} component bone facing surface** | **2^{nd} component bone cuts** |
|---|---|---|---|---|---|
| **Example: Femur** | Example: Femur | Example: Femur | Example: Tibia | Example: Tibia | Example: Tibia |
| **At least one bone cut** | Cartilage | Yes | Negative-match of 1^{st} component joint-facing (opposing cartilage) | At least one bone cut | Yes |
| **At least one bone cut** | Cartilage | Yes | Negative-match of 1^{st} component joint-facing (opposing cartilage) | Subchondral bone | Optional |
| **At least one bone cut** | Cartilage | Yes | Negative-match of 1^{st} component joint-facing (opposing cartilage) | Cartilage (same side, e.g. tibia) | Optional |
| **At least one bone cut** | Subchondral bone | Yes | Negative-match of 1^{st} component joint-facing (opposing subchondral bone) | At least one bone cut | Yes |
| **At least one bone cut** | Subchondral bone | Yes | Negative-match of 1^{st} component joint-facing (opposing subchondral bone) | Subchondral bone | Optional |
| **At least one bone cut** | Subchondral bone | Yes | Negative-match of 1^{st} component joint-facing (opposing subchondral bone) | Cartilage (same side, e.g. tibia) | Optional |
| **Subchondral bone** | Cartilage | Optional | Negative-match of 1^{st} component joint-facing (opposing cartilage) | At least one bone cut | Yes |
| **Subchondral bone** | Cartilage | Optional | Negative-match of 1^{st} component joint-facing (opposing cartilage) | Subchondral bone | Optional |
| **Subchondral bone** | Cartilage | Optional | Negative-match of 1^{st} component joint-facing (opposing cartilage) | Cartilage (same side, e.g. tibia) | Optional |
| **Subchondral bone** | Subchondral bone | Optional | Negative-match of 1^{st} component joint-facing (opposing subchondral bone) | At least one bone cut | Yes |
| **Subchondral bone** | Subchondral bone | Optional | Negative-match of 1^{st} component joint-facing (opposing subchondral bone) | Subchondral bone | Optional |
| **Subchondral bone** | Subchondral bone | Optional | Negative-match of 1^{st} component joint-facing (opposing subchondral bone) | Cartilage (same side, e.g. tibia) | Optional |
| **Subchondral bone** | Standard / Model | Optional | Negative-match of 1^{st} component joint-facing standard | At least one bone cut | Yes |
| **Subchondral bone** | Standard / Model | Optional | Negative-match of 1^{st} component joint-facing standard | Subchondral bone | Optional |
| **Subchondral bone** | Standard / Model | Optional | Negative-match of 1^{st} component joint-facing standard | Cartilage (same side, e.g. tibia) | Optional |
| **Subchondral bone** | Subchondral bone | Optional | Non-matching standard surface | At least one bone cut | Yes |
| **Subchondral bone** | Cartilage | Optional | Non-matching standard surface | At least one bone cut | Yes |

The disclosure can be applied to partial or total joint replacement systems. Bone cuts or changes to an implant component dimension described herein can be applied to a portion of the dimension, or to the entire dimension.

### Collecting and modeling patient-specific data

As mentioned above, disclosed is that implant components are designed and made using patient-specific data that is collected preoperatively. The patient-specific data can include points, surfaces, and/or landmarks, collectively referred to herein as "reference points." The reference points can be selected and used to derive a varied or altered surface, such as, without limitation, an ideal surface or structure. For example, the reference points can be used to create a model of the patient's relevant biological feature(s) and/or one or more patient-adapted surgical steps, tools, and implant components. For example the reference points can be used to design a patient-adapted implant component having at least one patient-specific or patient-engineered feature, such as a surface, dimension, or other feature.

Sets of reference points can be grouped to form reference structures used to create a model of a joint and/or an implant design. Designed implant surfaces can be derived from single reference points, triangles, polygons, or more complex surfaces or models of joint material, such as, for example, articular cartilage, subchondral bone, cortical bone, endosteal bone or bone marrow. Various reference points and reference structures can be selected and manipulated to derive a varied or altered surface, such as, without limitation, an ideal surface or structure.

The reference points can be located on or in the joint that will receive the patient-specific implant. For example, the reference points can include weight-bearing surfaces or locations in or on the joint, a cortex in the joint, and/or an endosteal surface of the joint. The reference points also can include surfaces or locations outside of but related to the joint. Specifically, reference points can include surfaces or locations functionally related to the joint. For example, in the knee joint, reference points can include one or more locations ranging from the hip down to the ankle or foot. The reference points also can include surfaces or locations homologous to the joint receiving the implant. For example, a knee, a hip, or a shoulder joint, reference points can include one or more surfaces or locations from the contralateral knee, hip, or shoulder joint.

Imaging data collected from the patient, for example, imaging data from one or more of x-ray imaging, digital tomosynthesis, cone beam CT, non-spiral or spiral CT, non-isotropic or isotropic MRI, SPECT, PET, ultrasound, laser imaging, photo-acoustic imaging, is used to qualitatively and/or quantitatively measure one or more of a patient's biological features, one or more of normal cartilage, diseased cartilage, a cartilage defect, an area of denuded cartilage, subchondral bone, cortical bone, endosteal bone, bone marrow, a ligament, a ligament attachment or origin, menisci, labrum, a joint capsule, articular structures, and/or voids or spaces between or within any of these structures.

The model that includes at least a portion of the patient's joint also can include or display, as part of the model, one or more resection cuts, one or more drill holes, (e.g., on a model of the patient's femur), and/or one or more implant components that have been designed for the particular patient using the model. Moreover, one or more resection cuts, one or more drill holes, and/or one or more implant components can be modeled and selected and/or designed separate from a model of a particular patient's biological feature.

### Modeling and addressing joint defects

Disclosed is that the reference points and/or measurements described above can be processed using mathematical functions to derive virtual, corrected features, which may represent a restored, ideal or desired feature from which a patient-adapted implant component can be designed. For example, one or more features, such as surfaces or dimensions of a biological structure can be modeled, altered, added to, changed, deformed, eliminated, corrected and/or otherwise manipulated (collectively referred to herein as "variation" of an existing surface or structure within the joint).

Variation of the joint or portions of the joint can includevariation of one or more external surfaces, internal surfaces, joint-facing surfaces, uncut surfaces, cut surfaces, altered surfaces, and/or partial surfaces as well as osteophytes, subchondral cysts, geodes or areas of eburnation, joint flattening, contour irregularity, and loss of normal shape. The surface or structure can be or reflect any surface or structure in the joint, including, bone surfaces, ridges, plateaus, cartilage surfaces, ligament surfaces, or other surfaces or structures. The surface or structure derived can be an approximation of a healthy joint surface or structure or can be another variation. The surface or structure can be made to include pathological alterations of the joint. The surface or structure also can be made whereby the pathological joint changes are virtually removed in whole or in part.

Once one or more reference points, measurements, structures, surfaces, models, or combinations thereof have been selected or derived, the resultant shape can be varied, deformed or corrected. Disclosed is that the variation can be used to select and/or design an implant component having an ideal or optimized feature or shape, e.g., corresponding to the deformed or corrected joint feature or shape. For example, the ideal or optimized implant shape reflects the shape of the patient's joint before he or she developed arthritis.

Alternatively or in addition, the variation can be used to select and/or design a patient-adapted surgical procedure to address the deformity or abnormality. For example, the variation can include surgical alterations to the joint, such as virtual resection cuts, virtual drill holes, virtual removal of osteophytes, and/or virtual building of structural support in the joint deemed necessary or beneficial to a desired final outcome for a patient.

### Osteophytes, subchondral voids, and other patient-specific defects

Corrections can be used to address osteophytes, subchondral voids, and other patient-specific defects or abnormalities. In the case of osteophytes, a design for the bone-facing surface of an implant component can be selected and/or designed after the osteophyte has been virtually removed. Alternatively, the osteophyte can be integrated into the shape of the bone-facing surface of the implant component **FIGS. 4A-4D** are exemplary drawings of an end of a femur **1010** having an osteophyte **1020.** In the selection and/or design of an implant component for a particular patient, an image or model of the patient's bone that includes the osteophyte can be transformed such that the osteophyte **1020** is virtually removed, as shown in **FIG. 4B** at removed osteophyte **1030,** to produce, as shown in **FIG. 4C****,** an implant component **1040** based on a smooth surface at the end of femur **1010.** Alternatively, as shown in **FIG. 4D****,** an implant component **1050** can be selected and/or designed to conform to the shape of the osteophyte **1020.** In the case of building additional or improved structure, additional features of the implant component then can be derived after bone-facing surface correction is modeled. Optionally, a surgical strategy and/or one or more guide tools can be selected and/or designed to reflect the correction and correspond to the implant component.

Similarly, to address a subchondral void, a selection and/or design for the bone-facing surface of an implant component can be derived after the void has been virtually removed (e.g., filled). Alternatively, the subchondral void can be integrated into the shape of the bone-facing surface of the implant component. **FIGS. 5A-5D** are exemplary drawings of an end of a femur **1110** having a subchondral void **1120.** During development of an implant, an image or model of the patient's bone that includes the void can be transformed such that the void **1120** is virtually removed, as shown in **FIG. 5B** at removed void **1130,** to produce, as shown in **FIG. 5C****,** an implant component **1140** based on a smooth surface at the end of femur **1110.** Alternatively, implant **1110** can be selected and/or designed to conform to the shape of void **1120,** as shown in **FIG. 5D****.** Note that, while virtually conforming to void **1120,** implant **1150** may not practically be able to be inserted into the void. Therefore, , the implant may only partially protrude into a void in the bone..

Certain embodiments described herein Disclosed is that collecting and using data from imaging tests to virtually determine in one or more planes one or more of an anatomic axis and a mechanical axis and the related misalignment of a patient's limb. The imaging tests that can be used to virtually determine a patient's axis and misalignment can include one or more of such as x-ray imaging, digital tomosynthesis, cone beam CT, non-spiral or spiral CT, non-isotropic or isotropic MRI, SPECT, PET, ultrasound, laser imaging, and photoacoustic imaging, including studies utilizing contrast agents. Data from these tests can be used to determine anatomic reference points or limb alignment, including alignment angles within the same and between different joints or to simulate normal limb alignment. Using the image data, one or more mechanical or anatomical axes, angles, planes or combinations thereof can be determined. Such axes, angles, and/or planes can include, or be derived from, one or more of a Whiteside's line, Blumensaat's line, transepicondylar line, femoral shaft axis, femoral neck axis, acetabular angle, lines tangent to the superior and inferior acetabular margin, lines tangent to the anterior or posterior acetabular margin, femoral shaft axis, tibial shaft axis, transmalleolar axis, posterior condylar line, tangent(s) to the trochlea of the knee joint, tangents to the medial or lateral patellar facet, lines tangent or perpendicular to the medial and lateral posterior condyles, lines tangent or perpendicular to a central weight-bearing zone of the medial and lateral femoral condyles, lines transecting the medial and lateral posterior condyles, for example through their respective centerpoints, lines tangent or perpendicular to the tibial tuberosity, lines vertical or at an angle to any of the aforementioned lines, and/or lines tangent to or intersecting the cortical bone of any bone adjacent to or enclosed in a joint. Moreover, estimating a mechanical axis, an angle, or plane also can be performed using image data obtained through two or more joints, such as the knee and ankle joint, for example, by using the femoral shaft axis and a centerpoint or other point in the ankle, such as a point between the malleoli

### Deformity correction and optimizing limb alignment

Information regarding the misalignment and the proper mechanical alignment of a patient's limb can be used to preoperatively design and/or select one or more features of a joint implant and/or implant procedure. For example, based on the difference between the patient's misalignment and the proper mechanical axis, a knee implant and implant procedure can be designed and/or selected preoperatively to include implant and/or resection dimensions that substantially realign the patient's limb to correct or improve a patient's alignment deformity. In addition, the process can include selecting and/or designing one or more surgical tools (e.g., guide tools or cutting jigs) to direct the clinician in resectioning the patient's bone in accordance with the preoperatively designed and/or selected resection dimensions.

Disclosed is that the degree of deformity correction that is necessary to establish a desired limb alignment is calculated based on information from the alignment of a virtual model of a patient's limb. The virtual model can be generated from patient-specific data, such 2D and/or 3D imaging data of the patient's limb. The deformity correction can correct varus or valgus alignment or antecurvatum or recurvatum alignment. Disclosed is that the desired deformity correction returns the leg to normal alignment, for example, a zero degree biomechanical axis in the coronal plane and absence of genu antecurvatum and recurvatum in the sagittal plane.

**FIG. 6** illustrates a coronal plane of the knee with exemplary resection cuts that can be used to correct lower limb alignment in a knee replacement. As shown in the figure, the selected and/or designed resection cuts can include different cuts on different portions of a patient's biological structure. For example, resection cut facets on medial and lateral femoral condyles can be non-coplanar and parallel **1602, 1602',** angled **1604, 1604',** or non-coplanar and non-parallel, for example, cuts **1602** and **1604'** or cuts **1602'** and **1604.** Similar, resection cut facets on medial and lateral portions of the tibia can be non-coplanar and parallel **1606, 1606',** angled and parallel **1608, 1608',** or non-coplanar and non-parallel, for example, cuts **1606** and **1608'** or cuts **1606'** and **1608.** Non-coplanar facets of resection cuts can include a step-cut **1610** to connect the non-coplanar resection facet surfaces. Selected and/or designed resection dimensions can be achieved using or more selected and/or designed guide tools (e.g., cutting jigs) that guide resectioning (e.g., guide cutting tools) of the patient's biological structure to yield the predetermined resection surface dimensions (e.g., resection surface(s), angles, and/or orientation(s). The bone-facing surfaces of the implant components can be designed to include one or more features (e.g., bone cut surface areas, perimeters, angles, and/or orientations) that substantially match one or more of the resection cut or cut facets that were predetermined to enhance the patient's alignment. As shown in **FIG. 6****,** certain combinations of resection cuts can aid in bringing the femoral mechanical axis **1612** and tibial mechanical axis **1614** into alignment **1616.**

Alternatively, or in addition, certain implant features, such as different implant thicknesses and/or surface curvatures across two different sides of the plane in which the mechanical axes **1612, 1614** are misaligned also can aid correcting limb alignment. For example, **FIG. 7** depicts a coronal plane of the knee shown with femoral implant medial and lateral condyles **1702, 1702'** having different thicknesses to help to correct limb alignment. These features can be used in combination with any of the resection cut **1704, 1704'** described above and/or in combination with different thicknesses on the corresponding portions of the tibial component. As described more fully below, independent tibial implant components and/or independent tibial inserts on medial and lateral sides of the tibial implant component can be used enhance alignment at a patient's knee joint. An implant component can include constant yet different thicknesses in two or more portions of the implant (e.g., a constant medial condyle thickness different from a constant lateral condyle thickness), a gradually increasing thickness across the implant or a portion of the implant, or a combination of constant and gradually increasing thicknesses.

### Preserving bone, cartilage or ligament

An implant component design or selection can depend, at least in part, on a threshold minimum implant component thickness. In turn, the threshold minimum implant component thickness can depend, at least in part, on patient-specific data, such as condylar width, femoral transepicondylar axis length, and/or the patient's specific weight. In this way, the threshold implant thickness, and/or any implant component feature, can be adapted to a particular patient based on a combination of patient-specific geometric data and on patient-specific anthropometric data. This approach can apply to any implant component feature for any joint, for example, the knee, the hip, or the shoulder.

### Ligament preservation

Implant design and modeling also can be used to achieve ligament sparing, for example, with regard to the PCL and/or the ACL. An imaging test can be utilized to identify, for example, the origin and/or the insertion of the PCL and the ACL on the femur and tibia. The origin and the insertion can be identified by visualizing, for example, the ligaments directly, as is possible with MRI or spiral CT arthrography, or by visualizing bony landmarks known to be the origin or insertion of the ligament such as the medial and lateral tibial spines.

An implant system can then be selected or designed based on the image data so that, for example, the femoral component preserves the ACL and/or PCL origin, and the tibial component preserves the ACL and/or PCL attachment. The implant can be selected or designed so that bone cuts adjacent to the ACL or PCL attachment or origin do not weaken the bone to induce a potential fracture.

For ACL preservation, the implant can have two unicompartmental tibial components that can be selected or designed and placed using the image data. Alternatively, the implant can have an anterior bridge component. The width of the anterior bridge in AP dimension, its thickness in the superoinferior dimension or its length in mediolateral dimension can be designed using the imaging data and, specifically, the known insertion of the ACL and/or PCL.

### Establishing normal or near-normal joint kinematics

In certain embodiments, bone cuts and iImplant shape including at least one of a bone-facing or a joint-facing surface of the implant can be designed or selected to achieve normal joint kinematics.

Disclosed is a computer program simulating biomotion of one or more joints, such as, for example, a knee joint, or a knee and ankle joint, or a hip, knee and/or ankle joint can be utilized. Patient-specific imaging data can be fed into this computer program. For example, a series of two-dimensional images of a patient's knee joint or a three-dimensional representation of a patient's knee joint can be entered into the program. Additionally, two-dimensional images or a three-dimensional representation of the patient's ankle joint and/or hip joint may be added.

Optionally, other data including anthropometric data may be added for each patient. These data is selected from patient's age, gender, weight, height, size, body mass index, and race, desired limb alignment or deformity correction the model. The position of bone cuts on one or more articular surfaces as well as the intended location of implant bearing surfaces on one or more articular surfaces can be entered into the model.

A patient-specific biomotion model can be derived that includes combinations of parameters listed above. The biomotion model can simulate various activities of daily life including normal gait, stair climbing, descending stairs, running, kneeling, squatting, sitting and any other physical activity. The biomotion model can start out with standardized activities, typically derived from reference databases. These reference databases can be, for example, generated using biomotion measurements using force plates and motion trackers using radiofrequency or optical markers and video equipment.

The biomotion model can then be individualized with use of patient-specific information selected from the patient's age, gender, weight, height, body mass index, and race, the desired limb alignment or deformity correction, and the patient's imaging data, for example, a series of two-dimensional images or a three-dimensional representation of the joint for which surgery is contemplated.

An implant shape including associated bone cuts generated in the preceding optimizations, for example, limb alignment, deformity correction, bone preservation on one or more articular surfaces, can be introduced into the model. **Table 2** includes an exemplary list of parameters that can be measured in a patient-specific biomotion model.

**Table 2: Parameters measured in a patient-specific biomotion model for various implants**

| **Joint implant** | **Measured Parameter** |
|---|---|
| **knee** | Medial femoral rollback during flexion |
| **knee** | Lateral femoral rollback during flexion |
| **knee** | Patellar position, medial, lateral, superior, inferior for different flexion and extension angles |
| **knee** | Internal and external rotation of one or more femoral condyles |
| **knee** | Internal and external rotation of the tibia |
| **knee** | Flexion and extension angles of one or more articular surfaces |
| **knee** | Anterior slide and posterior slide of at least one of the medial and lateral femoral condyles during flexion or extension |
| **knee** | Medial and lateral laxity throughout the range of motion |
| **knee** | Contact pressure or forces on at least one or more articular surfaces, e.g. a femoral condyle and a tibial plateau, a trochlea and a patella |
| **knee** | Contact area on at least one or more articular surfaces, e.g. a femoral condyle and a tibial plateau, a trochlea and a patella |
| **knee** | Forces between the bone-facing surface of the implant, an optional cement interface and the adjacent bone or bone marrow, measured at least one or multiple bone cut or bone-facing surface of the implant on at least one or multiple articular surfaces or implant components. |
| **knee** | Ligament location, e.g. ACL, PCL, MCL, LCL, retinacula, joint capsule, estimated or derived, for example using an imaging test. |
| **knee** | Ligament tension, strain, shear force, estimated failure forces, loads for example for different angles of flexion, extension, rotation, abduction, adduction, with the different positions or movements optionally simulated in a virtual environment. |
| **knee** | Potential implant impingement on other articular structures, e.g. in high flexion, high extension, internal or external rotation, abduction or adduction or any combinations thereof or other angles / positions / movements. |
| **Hip, shoulder or other joint** | Internal and external rotation of one or more articular surfaces |
| **Hip, shoulder or other joint** | Flexion and extension angles of one or more articular surfaces |
| **Hip, shoulder or other joint** | Anterior slide and posterior slide of at least one or more articular surfaces during flexion or extension, abduction or adduction, elevation, internal or external rotation |
| **Hip, shoulder or other joint** | Joint laxity throughout the range of motion |
| **Hip, shoulder or other joint** | Contact pressure or forces on at least one or more articular surfaces, e.g. an acetabulum and a femoral head, a glenoid and a humeral head |
| **Hip, shoulder or other joint** | Forces between the bone-facing surface of the implant, an optional cement interface and the adjacent bone or bone marrow, measured at least one or multiple bone cut or bone-facing surface of the implant on at least one or multiple articular surfaces or implant components. |
| **Hip, shoulder or other joint** | Ligament location, e.g. transverse ligament, glenohumeral ligaments, retinacula, joint capsule, estimated or derived, for example using an imaging test. |
| **Hip, shoulder or other joint** | Ligament tension, strain, shear force, estimated failure forces, loads for example for different angles of flexion, extension, rotation, abduction, adduction, with the different positions or movements optionally simulated in a virtual environment. |
| **Hip, shoulder or other joint** | Potential implant impingement on other articular structures, e.g. in high flexion, high extension, internal or external rotation, abduction or adduction or elevation or any combinations thereof or other angles / positions / movements. |

- The above list is exemplary.
- The resultant biomotion data can be used to further optimize the implant design with the objective to establish normal or near normal kinematics. The implant optimizations can include one or multiple implant components. Implant optimizations based on patient-specific data including image based biomotion data include,
- Changes to external, joint-facing implant shape in coronal plane
- Changes to external, joint-facing implant shape in sagittal plane
- Changes to external, joint-facing implant shape in axial plane
- Changes to external, joint-facing implant shape in multiple planes or three dimensions
- Changes to internal, bone-facing implant shape in coronal plane
- Changes to internal, bone-facing implant shape in sagittal plane
- Changes to internal, bone-facing implant shape in axial plane
- Changes to internal, bone-facing implant shape in multiple planes or three dimensions
- Changes to one or more bone cuts, for example with regard to depth of cut, orientation of cut

Any single one or combinations of the above or all of the above on at least one articular surface or implant component or multiple articular surfaces or implant components.

When changes are made on multiple articular surfaces or implant components, these can be made in reference to or linked to each other. For example, in the knee, a change made to a femoral bone cut based on patient-specific biomotion data can be referenced to or linked with a concomitant change to a bone cut on an opposing tibial surface, for example, if less femoral bone is resected, the computer program may elect to resect more tibial bone.

Similarly, if a femoral implant shape is changed, for example on an external surface, this can be accompanied by a change in the tibial component shape. This is, for example, particularly applicable when at least portions of the tibial bearing surface negatively-match the femoral joint-facing surface.

Similarly, if the footprint of a femoral implant is broadened, this can be accompanied by a widening of the bearing surface of a tibial component. Similarly, if a tibial implant shape is changed, for example on an external surface, this can be accompanied by a change in the femoral component shape. This is, for example, particularly applicable when at least portions of the femoral bearing surface negatively-match the tibial joint-facing surface.

Similarly, if a patellar component radius is widened, this can be accompanied by a widening of an opposing trochlear bearing surface radius, or vice-versa.

Any combination is possible as it pertains to the shape, orientation, and size of implant components on two or more opposing surfaces.

By optimizing implant shape in this manner, it is possible to establish normal or near normal kinematics. Moreover, it is possible to avoid implant related complications, including but not limited to anterior notching, notch impingement, posterior femoral component impingement in high flexion, and other complications associated with existing implant designs.

Biomotion models for a particular patient can be supplemented with patient-specific finite element modeling or other biomechanical models known in the art. Resultant forces in the knee joint can be calculated for each component for each specific patient. The implant can be engineered to the patient's load and force demands. For instance, a 1251b. patient may not need a tibial plateau as thick as a patient with 280 lbs. Similarly, the polyethylene can be adjusted in shape, thickness and material properties for each patient. For example, a 3 mm polyethylene insert can be used in a light patient with low force and a heavier or more active patient may need an 8mm polymer insert or similar device.

### Designing an implant component

The assessment process includes selecting and/or designing one or more features and/or feature measurements of an implant component that is adapted (e.g., patient-adapted based on one or more of a particular patient's biological features and/or feature measurements) to achieve or address, at least in part, one or more of the following parameters for the particular patient: (1) correction of a joint deformity; (2) correction of a limb alignment deformity; (3) preservation of bone, cartilage, and/or ligaments at the joint; (5) preservation, restoration, or enhancement of joint kinematics, including, for example, ligament function and implant impingement; and (7) preservation, restoration, or enhancement of other target features.

Preserving, restoring, or enhancing bone, cartilage, and/or ligaments can include, for example, identifying diseased tissue from one or more images of the patient's joint, identifying a minimum implant thickness for the patient (based on, for example, femur and/or condyle size and patient weight); virtually assessing combinations of resection cuts and implant component features, such as variable implant thickness, bone cut numbers, bone cut angles, and/or bone cut orientations; identifying a combination of resection cuts and/or implant component features that, for example, remove diseased tissue and also provide maximum bone preservation (i.e., minimum amount of resected bone) and at least the minimum implant thickness for the particular patient; one or more implant components to provide implant component features that provide removal of the diseased tissue, maximum bone preservation, and at least the minimum implant thickness for the particular patient.

The assessment process can be iterative in nature. For example, one or more first parameters can be assessed and the related implant component features and feature measurements tentatively or conditionally can be determined. Next, one or more second parameters can be assessed and, optionally, one or more features and/or feature measurements determined. Then, the tentative or conditional features and/or feature measurements for the first assessed parameter(s) optionally can be altered based on the assessment and optional determinations for the second assessed parameters. The assessment process can be fully automated or it can be partially automated allowing for user interaction. User interaction can be particularly useful for quality assurance purposes.

In the assessment, different weighting can be applied to any of the parameters or parameter thresholds, for example, based on the patient's age, the surgeon's preference or the patient's preference. Feedback mechanisms can be used to show the user or the software the effect that certain feature and/or feature measurement changes can have on desired changes to parameters values, e.g., relative to selected parameter thresholds. For example, a feedback mechanism can be used to determine the effect that changes in features intended to maximize bone preservation (e.g., implant component thickness(es), bone cut number, cut angles, cut orientations, and related resection cut number, angles, and orientations) have on other parameters such as limb alignment, deformity correction, and/or joint kinematic parameters, for example, relative to selected parameter thresholds.

### Setting and weighing parameters

As described herein, certain embodiments Disclosed are aspects that can apply modeling, for example, virtual modeling and/or mathematical modeling, to identify optimum implant component features and measurements, to achieve or advance one or more parameter targets or thresholds. For example, a model of patient's joint or limb can be used to identify, select, and/or design one or more optimum features and/or feature measurements relative to selected parameters for an implant component. Disclosed is that a physician, clinician, or other user can select one or more parameters, parameter thresholds or targets, and/or relative weightings for the parameters included in the model. Alternatively or in addition, clinical data, for example obtained from clinical trials, or intraoperative data, can be included in selecting parameter targets or thresholds, and/or in determining optimum features and/or feature measurements for an implant component.

### Generating bone cuts and resected surfaces

### Libraries

Disclosed are implants of various sizes, shapes, curvatures and thicknesses with various types and locations and orientations and number of bone cuts can be selected and/or designed and manufactured. The implant designs can be selected from, catalogued in, and/or stored in a library. The library can be a virtual library of implants, or components, or component features that can be combined and/or altered to create a final implant. The library can include a catalogue of physical implant components. Physical implant components can be identified and selected using the library. The library can include previously-generated implant components having one or more patient-adapted features, and/or components with standard or blank features that can be altered to be patient-adapted. Accordingly, implants and/or implant features can be selected from the library.

Alternatively or in addition, one or more features of an implant component and/or implant procedure can be preoperatively derived from patient-specific data to provide a patient-engineered feature, for example, to optimize one or more parameters, such as one or more of the parameters described above. A bone preserving femoral implant component can include an inner, bone-facing surface (i.e., superior surface) having one or bone cuts that, at least in part, are patient-derived, optionally together with matching patient-derived resection cuts, to minimize the amount of resected bone (and maximize the amount of retained bone), for example, on the patient's femur. This patient-engineered implant component feature can be preoperatively selected and/or designed based on the patient's joint dimensions as seen, for example, on a series of two-dimensional images or a three-dimensional representation generated, for example, from a CT scan or MRI scan.

### Examples

**Examples not falling within the scopes of the claims are for illustrative purposes only.**

### Example 1: Exemplary design process for certain patient-specific total knee implants

**Example 1** describes an exemplary process for designing a patient-adapted implant component. **Example 2** describes an exemplary patient-adapted knee implants components and methods for designing the same. **Example 3** describes exemplary knee implants components having patient-adapted features and non-traditional features

This example describes an exemplary process for selecting and/or designing a patient-adapted total knee implant, for example, a knee implant having one or more patient-specific and/or patient-engineered based on patient-specific data. The steps described in this process can be performed in any order and can be performed more than once in a particular process. For example, one or more steps can be reiterated and refined a second, third, or more times, before, during, or after performing other steps or sets of steps in the process. While this process specifically describes steps for selecting and/or designing a patient-specific total knee implant, it can be adapted to design for example, patient-adapted bicompartmental knee implants, unicompartmental knee implants, and implants for shoulders and hips, vertebrae, and other joints.

### Methods

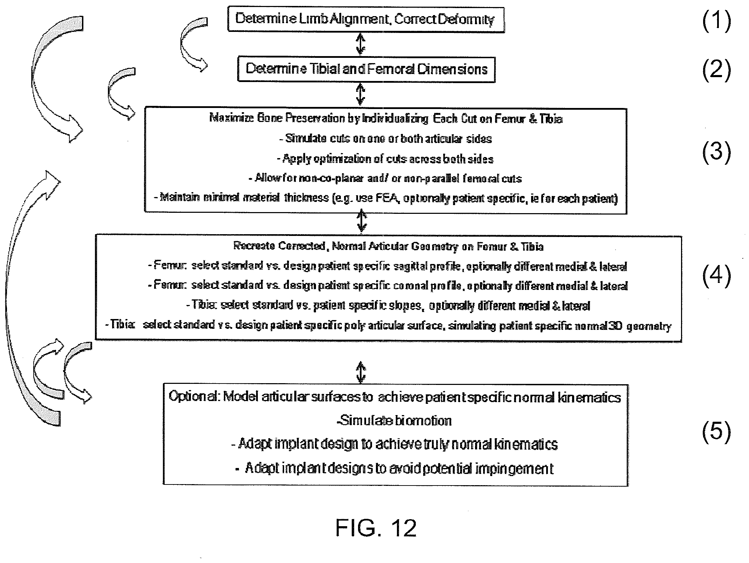

The exemplary process shown in **FIG. 12** includes four general steps and, optionally, can include a fifth general step. Each general step includes various specific steps. The general steps are identified as (1)-(5) in the figure. These steps can be performed virtually, for example, by using one or more computers that have or can receive patient-specific data and specifically configured software or instructions to perform such steps.

In general step (1), limb alignment and deformity corrections are determined, to the extent that either is needed for a specific patient's situation. In general step (2), the requisite tibial and femoral dimensions of the implant components are determined based on patient-specific data obtained, for example, from image data of the patient's knee.

In general step (3), bone preservation is maximized by virtually determining a resection cut strategy for the patient's femur and/or tibia that provides minimal bone loss optionally while also meeting other user-defined parameters such as, for example, maintaining a minimum implant thickness, using certain resection cuts to help correct the patient's misalignment, removing diseased or undesired portions of the patient's bone or anatomy, and/or other parameters. This general step can include one or more of the steps of (i) simulating resection cuts on one or both articular sides (e.g., on the femur and/or tibia), (ii) applying optimized cuts across one or both articular sides, (iii) allowing for non-co-planar and/or non-parallel femoral resection cuts (e.g., on medial and lateral corresponding portions of the femur) and, optionally, non-co-planar and/or non-parallel tibial resection cuts (e.g., on medial and lateral corresponding portions of the tibia), and (iv) maintaining and/or determining minimal material thickness. The minimal material thickness for the implant selection and/or design can be an established threshold, for example, as previously determined by a finite element analysis ("FEA") of the implant's standard characteristics and features. Alternatively, the minimal material thickness can be determined for the specific implant, for example, as determined by an FEA of the implant's standard and patient-specific characteristics and features. This step identifies for a surgeon the bone resection design to perform in the surgical theater and it also identifies the design of the bone-facing surface(s) of the implant components, which substantially negatively-match the patient's resected bone surfaces, at least in part.

In general step (4), a corrected, normal and/or optimized articular geometry on the femur and tibia is recreated virtually. For the femur, this general step can include, for example, the step of: (i) selecting a standard sagittal profile, or selecting and/or designing a patient-engineered or patient-specific sagittal profile; and (ii) selecting a standard coronal profile, or selecting and/or designing a patient-specific or patient-engineered coronal profile. Optionally, the sagittal and/or coronal profiles of one or more corresponding medial and lateral portions (e.g., medial and lateral condyles) can include different curvatures. For the tibia, this general step includes one or both of the steps of: (iii) selecting a standard anterior-posterior slope, and/or selecting and/or designing a patient-specific or patient-engineered anterior-posterior slope, either of which optionally can vary from medial to lateral sides; and (iv) selecting a standard poly-articular surface, or selecting and/or designing a patient-specific or patient-engineered poly-articular surface. The patient-specific poly-articular surface can be selected and/or designed, for example, to simulate the normal or optimized three-dimensional geometry of the patient's tibial articular surface. The patient-engineered poly-articular surface can be selected and/or designed, for example, to optimize kinematics with the bearing surfaces of the femoral implant component. This step can be used to define the bearing portion of the outer, joint-facing surfaces (i.e., articular surfaces) of the implant components.

In optional general step (5), a virtual implant model (for example, generated and displayed using a computer specifically configured with software and/or instructions to assess and display such models) is assessed and can be altered to achieve normal or optimized kinematics for the patient. For example, the outer joint-facing or articular surface(s) of one or more implant components can be assessed and adapted to improve kinematics for the patient. This general step can include one or more of the steps of: (i) virtually simulating biomotion of the model, (ii) adapting the implant design to achieve normal or optimized kinematics for the patient, and (iii) adapting the implant design to avoid potential impingement.

### Results and discussion

The exemplary process described above yields both a predetermined surgical resection design for altering articular surfaces of a patient's bones during surgery and a design for an implant that specifically fits the patient, for example, following the surgical bone resectioning. Specifically, the implant selection and/or design, which can include manufacturing or machining the implant to the selected and/or designed specifications using known techniques, includes one or more patient-engineered bone-facing surfaces that negatively-match the patient's resected bone surface. The implant also can include other features that are patient-adapted, such as minimal implant thickness, articular geometry, and kinematic design features. This process can be applied to various joint implants and to various types of joint implants. For example, this design process can be applied to a total knee, cruciate retaining, posterior stabilized, and/or ACL/PCL retaining knee implants, bicompartmental knee implants, unicompartmental knee implants, and other joint implants, for example, for the shoulder, hip, elbow, spine, or other joints.

The exemplary process described above, including the resulting patient-adapted implants and predetermined bone resectioning design, offers several advantages over traditional primary and revision implants and related processes. For example, it allows for one or more pre-primary implants such that a subsequent replacement or improvement can take the form of a primary implant. Specifically, because the process described herein can minimize the amount of bone that is resected, enough bone stock may remain such that a subsequent procedure may be performed with a traditional, primary, off-the-shelf implant. This offers a significant advantage for younger patients who may require in their lifetime more than a single revision for an implant. In fact, the exemplary process described above may allow for two or more pre-primary implants or procedures before so much bone stock is sacrificed that a traditional, primary implant is required.

The advantageous minimal bone resectioning and therefore minimal bone loss that is achieved with this process arises from the fact that the bone-facing surfaces of the implants are derived for each patient based on patient-specific data, such as, for example, data derived from images of the patient's joint, size or weight of the patient, size of the joint, and size, shape and/or severity of defects and/or disease in the joint. This patient-adapted approach allows for the bone-facing surface of the implant components to be optimized with respect to any number of parameters, including minimizing bone loss, using any number of resection cuts and corresponding implant component bone cuts and bone cut facets to conserve bone for the patient. With traditional implants, the implant's bone-facing surface includes standard bone cuts and the resection cuts to the patient's bone are made to fit those standard bone cuts.

Another advantage to this process is that the selection and/or design process can incorporate any number of target parameters such that any number of implant component features and resection cuts can be selected and/or designed to meet one or more parameters that are predetermined to have clinical value. For example, in addition to bone preservation, a selection and/or design process can include target parameters to restore a patient's native, normal kinematics, or to provide optimized kinematics. For example, the process for selecting and/or designing an implant and/or resection cuts can include target parameters such as reducing or eliminating the patient's mid-flexion instability, reducing or eliminating "tight" closure, improving or extending flexion, improving or restoring cosmetic appearance, and/or creating or improving normal or expected sensations in the patient's knee. The design for a tibial implant can provide an engineered surface that replicates the patient's normal anatomy yet also allows for low contact stress on the tibia.

For surgeons and medical professionals, this process also provides a simplified surgical technique. The selected and/or designed bone cuts and, optionally, other features that provide a patient-adapted fit for the implant components eliminates the complications that arise in the surgical setting with traditional, misfitting implants. Moreover, since the process and implant component features are predetermined prior to surgery, model images of the surgical steps can be provided to the surgeon as a guide.

As noted above, the design of an implant component can include manufacturing or machining the component in accordance with the implant design specifications. Manufacturing can include, for example, using a designed mold to form the implant component. Machining can include, for example, altering a selected blank form to conform to the implant design specifications. For example, using the steps described above, the femoral implant component can be manufactured from a designed mold and the tibial implant component, including each of a tibial tray and insert, can be customized from a selected starting tray and insert, for example, from blanks.

### Example 2: Patient-adapted femoral implant component with five bone cuts and corresponding resection cuts

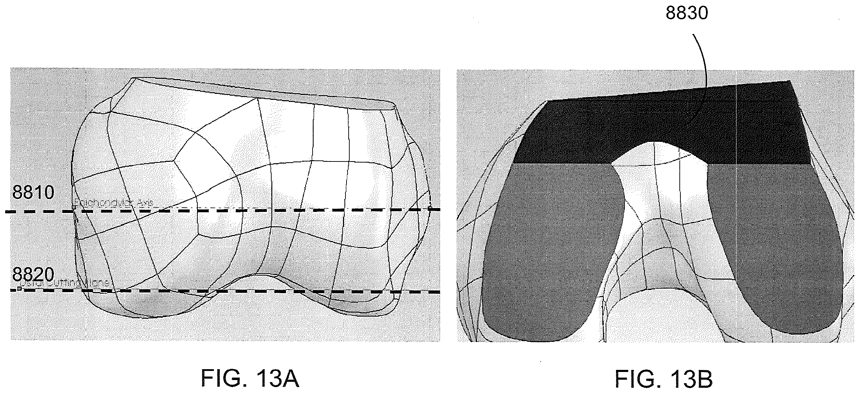

This example describes two exemplary methods for designing resection cuts to a patient's femur and related bone cuts on the bone-facing surface of a femoral implant component designed for the patient. In particular, in both methods, a model of a patient's distal femur is created based on data from patient-specific two- or three-dimensional images of the patient's femur. As shown in **FIG. 13A****,** the epicondylar axis **8810** is determined for the patient's femur. Then, the resection cut planes and cut angles (and corresponding implant component cut planes and cut angles) are assessed and selected using the epicondylar axis **8810** as a reference. Specifically, four of the five cut planes - the distal cut, posterior cut, posterior chamfer cut, and anterior chamfer cut - are designed to be parallel with the epicondylar axis **8810.** **FIG. 13A** shows the distal cut plane **8820** parallel to the epicondylar axis **8810.** However, for the particular patient, the anterior cut plane is designed to be oblique to the epicondylar axis **8810,** which can minimize the amount of bone resected on the lateral side of the cut. **FIG. 13B** shows an example of an anterior oblique cut plane **8830.**

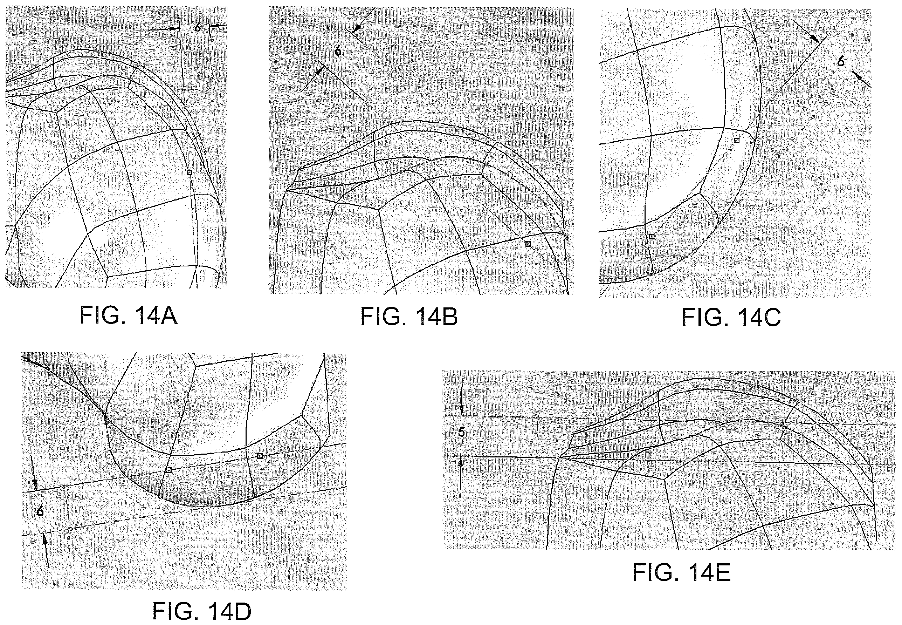

For each of the five cut planes, optimized cuts (i.e., resection cuts) tangent to the bone surface at the angle of each resection plane also is determined. The optimized cuts as shown in **FIGS. 14A** to **14E** included a maximum cut depth of 6 mm for the distal cut plane **(****FIG. 14A****),** the anterior chamfer cut plane **(****FIG. 14B****),** the posterior chamfer cut **(****FIG. 14C****),** and the posterior cut plane **(FIG. 14D).** The maximum cut depth is 5 mm for the anterior cut plane **(****FIG. 14E****).** Optimized cuts can be determined based on one or more parameters, including those described above. In this example, optimized cut were determined, at least in part, to minimize resected bone while providing greater than a threshold minimum implant thickness. Deeper resection cuts allow for a thicker implant, but require greater bone loss. Typically, the thinnest resection cut depth and, accordingly, the minimum implant thickness occurs at the intersections between cut planes. Accordingly, alternatively or in addition to altering cut plane depths, the number of cut planes, the cut plane angles and/or the cut plane orientations can be altered to provide deeper cut plane intersections and corresponding greater minimum implant thickness at the bone cut intersections while also minimizing the amount of bone resected from the patient.

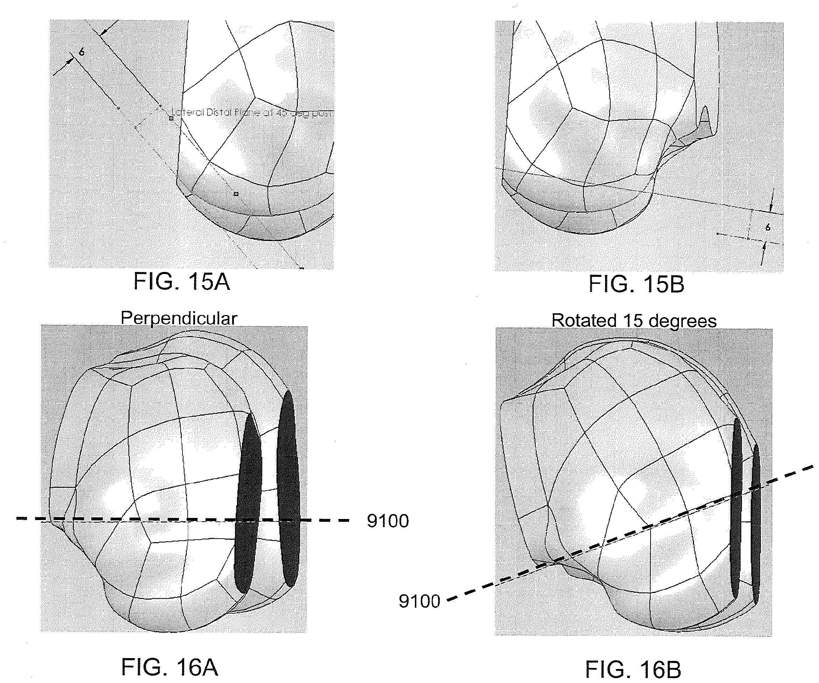

The optimized number of cut planes, depths of cut planes, angles of cut planes and/or orientations of cut planes can be determined independently for each of the medial and lateral femoral condyles. For example, **FIGS. 14A** to **14E** show optimized cut planes based on the medial condyle. However, **FIGS. 15A** and **15B** show cut planes for the lateral condyle posterior chamfer **(****FIG. 15A****)** and lateral condyle posterior **(****FIG. 15B****)** cut planes that are independently optimized (i.e., relative to the medial condyle posterior chamfer and medial condyle posterior cut planes, respectively) based on patient-specific data for the lateral condyle. This type of independent optimization between condyles can result in a different number of cut plane facets, different angles of cut plane facets, and/or different depths of cut plane facets on corresponding portions of medial and lateral condyles.

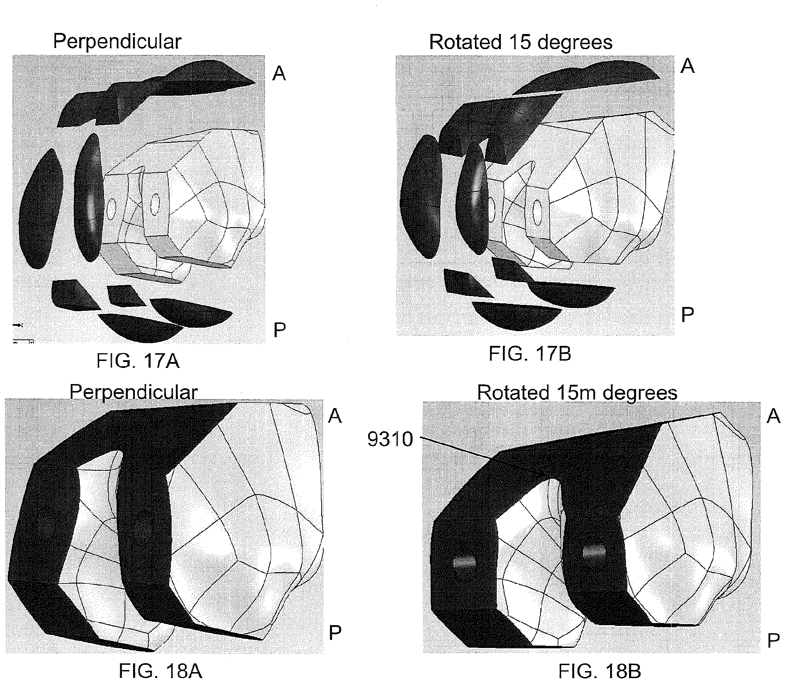

Two exemplary resection cut designs (and corresponding implant component bone cut design e.g., that includes substantially matching cut features of the resection cut design) is based on five cut planes are described in this example. In the first design, shown in **FIG. 16A****,** a distal cut plane is designed perpendicular to the sagittal femoral axis **9100.** In the second design, referred to as a "flexed" or "flex-fit" design" and shown in **FIG. 16B****,** the distal cut plane is rotated 15 degrees in flexion from the perpendicular to the sagittal femoral axis. The additional four cut planes are shifted accordingly for each design method, as shown in **FIGS. 17A** and **17B****.**

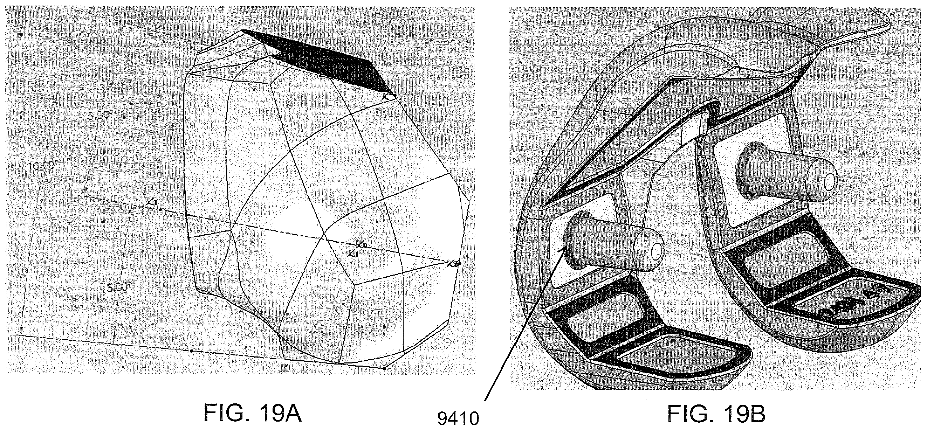

**FIGS. 18A** and **18B** show the completed cut femur models for each cut design. For each design, the maximum resection depth for each cut plane was 6 mm, except for the anterior cut plane, which was 5 mm. The "flex-fit" design can provide more posterior coverage in high flexion. However, it also may require more anterior bone resectioning to achieve sufficient coverage and may require particular attention during actual bone cutting to avoid any incomplete bone removal at the trochlear notch **9310.** In certain aspects of a cut plane design, the anterior and posterior cut planes diverge from the component peg axis by five degrees each, as shown in **FIG. 19A****.** With a traditional femoral implant component, the posterior and anterior cut planes diverge 2 degrees and 7 degrees, respectively, from the peg axis. Moreover, in certain aspects, the peg can be designed to have various dimensions. For example, the design in **FIG. 19B** includes a peg diameter of 7 mm tapering to about 6.5 mm, a length of 14 mm with a rounded tip, and a base with a 1 mm fillet **9410.**

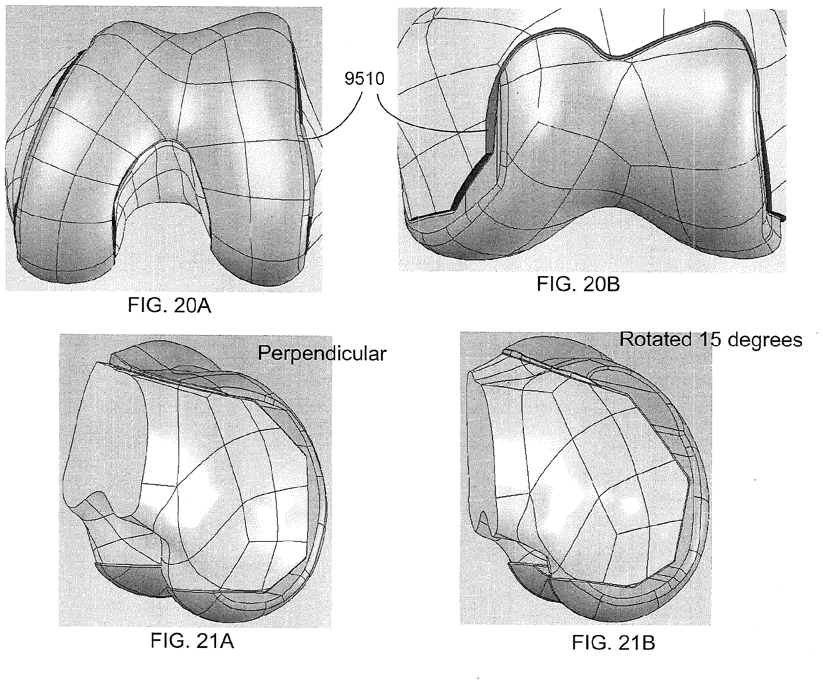

An exemplary bone facing surface of the femoral implant component design is shown in **FIGS. 20A** and **20B****.** In addition to optimized cut planes described above, these implant components also include a patient-adapted peripheral margin **9510** that is 0.5 mm from the edge of cut bone. The designs also can include engineered coronal curvatures on the condyles. Side views of the resulting femoral implant component designs for the first and second design methods are shown in **FIGS. 21A** and **21B****.** This sagittal view of the implant components shows the difference in anterior and posterior coverage for the two designs. As seen by a comparison of the two figures, the flexed cut design provides greater posterior coverage, which enhances deep knee flexion for the patient. Accordingly, as shown by this example, one or more features or measurements derived from patient-specific data are used to preoperatively select and/or design implant component features that target and achieve more than one parameter, in this case preservation of the patient's bone and preservation, restoration, or enhancement of the patient's joint kinematics.

As mentioned above, the optimization of resection cuts and implant component bone cuts can result in a cut design that has any number of cut planes or facets, depths of cut planes or facets, angles of cut planes or facets, and/or orientations of cut planes or facets. In addition to optimizing the cut planes to minimize bone loss and maximize implant thickness, various other parameters can be included in the cut plane optimization. In this example, the flexed cut design was used to help preserve, restore, or enhance the patient's joint kinematics. Additional parameters that may be included in the determination of optimized resection and bone cuts can include, but are not limited to, one or more of: (1) deformity correction and/or limb alignment (2) maximizing preservation of cartilage, or ligaments, (3) maximizing preservation and/or optimization of other features of the patient's anatomy, such as trochlea and trochlear shape, (4) further restoration and/or optimization of joint kinematics or biomechanics (5) restoration or optimization of joint-line location and/or joint gap width, and (6) preservation, restoration, or enhancement of other target features.

### Example 3: Design of a femoral component of a total knee replacement with a bone-facing surface that optimizes bone preservation

This example describes an exemplary design of femoral implant component. In particular, the femoral component includes seven bone cuts on its inner, bone-facing surface.

### Methods

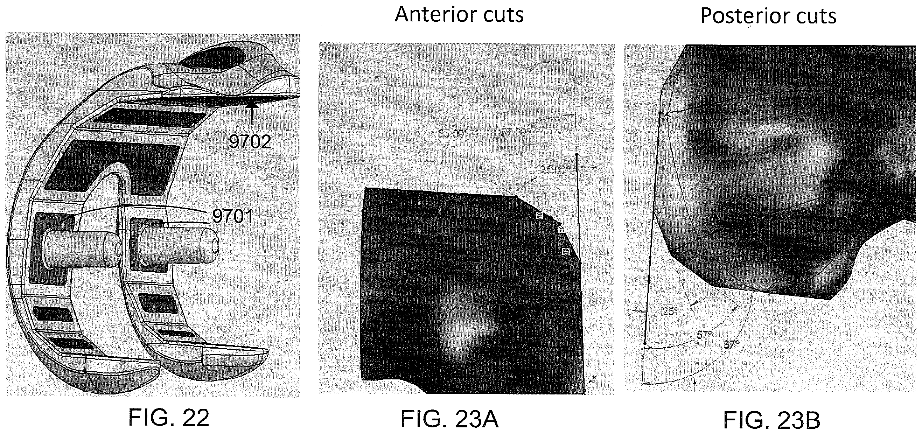

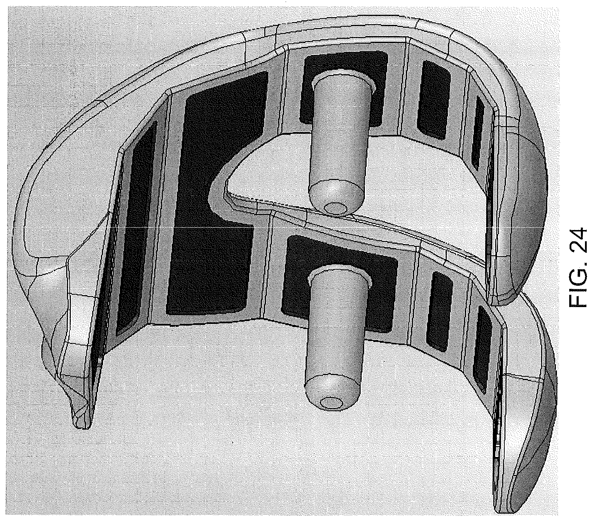

A femoral implant component (PCL-retaining) is designed with seven bone cuts for a femur-first implantation technique. The design of the implant component is depicted in **FIG. 22****.** The seven bone cuts on the inner, bone-facing surface of the implant component include a distal bone cut **9701** (including lateral and medial facets) that is perpendicular to the sagittal femoral axis, and an anterior cut **9702.** The corresponding resection cut planes are shown in **FIG. 23A** and in **FIG. 23B****.** Specifically, a first anterior chamfer cut plane is at 25 degrees, a second anterior chamfer cut plane is at 57 degrees, and an anterior cut plane is at 85 degrees relative to the distal femoral cut plane, as shown in **FIG. 23A****.** Moreover, a first posterior chamfer cut plane is at 25 degrees, a second posterior chamfer cut plane is at 57 degrees, and a posterior chamfer cut plane is at 87 degrees relative to the distal femoral cut plane, as shown in **FIG. 23B****.** The femoral implant includes bone cuts that substantially negatively-match (e.g., in cut angle, area, and/or orientation) the resection cuts on these cut planes. The femoral implant component also can include on its bone-facing surface cement cutouts 9704 that are 0.5 mm deep and offset from the outer edge by 2 mm, and a peg protruding from the each of the lateral and medial distal bone cuts facets. The pegs are 7 mm in diameter, 17 mm long and are tapered by 0.5 degrees as they extend from the component. **FIG. 24** shows the cement pocket and peg features.

### Results and Discussion

In a traditional femoral implant component, the bone-facing surface consists of five standard bone cuts. However, the femoral component in this example includes seven bone cuts on the bone-facing surface. The additional bone cuts can allow for the corresponding resection cut planes be less deep from the bone surface to insure that the cut plane intersections have a depth below the bone surface that allows for a minimum implant thickness. Accordingly, less bone can be resected for a seven-bone-cut implant component than for a traditional five-bone-cut implant component to provide the same minimum implant component thickness, e.g., at the bone cut intersections. Moreover, the outer, joint-facing surface of the implant component described in this example includes a combination of patient-adapted features and standard features.

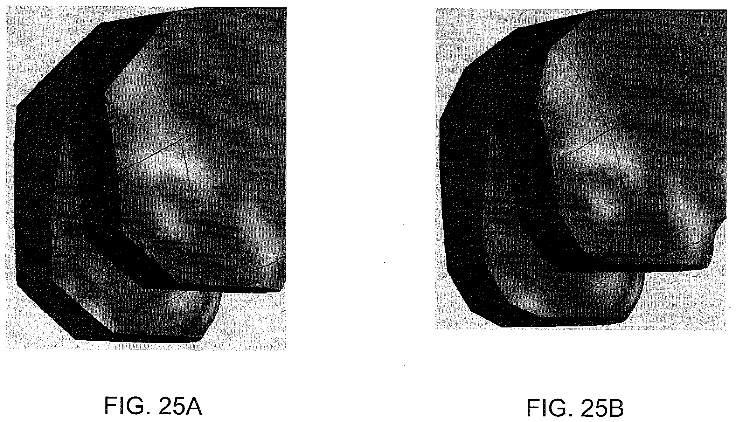

**FIG. 25A** shows a five-cut-plane femoral resection design for a femoral implant component having five bone cuts. **FIG. 25B** shows a seven-cut-plane femoral resection design for a femoral implant component having seven bone cuts. Each cut design was performed on the same patient femur model (i.e., having identical bone volumes). In addition, the corresponding five-bone-cut implant component and seven-bone-cut implant component were both designed meet or exceed the same minimum implant thickness. After performing the resection cuts, the model of the patient's femur having five resection cuts retained bone volume of 103,034 mm3, while the model of the patient's femur having seven bone cuts retained a bone volume of 104,220 mm3. As this analysis shows, the seven-bone-cut implant component saved substantially more of the patient's bone stock, in this case more than 1,000 mm³, as compared to the five-bone cut implant component.

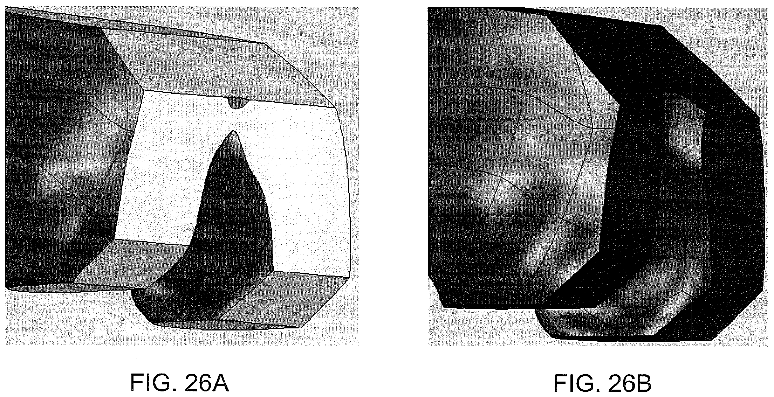

A similar analysis was performed to assess relative bone loss between a five-cut design and a five-flexed cut design. **FIG. 26A** shows a patient's femur having five, not flexed resection cuts and **FIG. 26B** shows the same femur but with five, flexed resection cuts. As shown, the model having five, not flexed resection cuts retains a bone volume of 109,472 mm³, while the model having five, flexed resection cuts retains a bone volume of 105,760 mm³. As this analysis shows, the not-flexed-five-bone-cut implant component saved substantially more of the patient's bone stock, in this case nearly 4,000 mm³, as compared to the flexed-five-bone-cut cut implant component. However, as noted in **Example 2,** the flexed cut design can offer other advantages, such as greater posterior coverage and enhanced deep-knee flexion, which can be weighed relative to all selected parameters and accordingly integrated in the selection and/or design of an implant component.

FIGS. 27A to 27D show outlines of a traditional five-cut femoral component (in hatched lines) overlaid with, in **27A,** a femur having seven optimized resection cuts for matching an optimized seven-bone-cut implant component; in **FIG. 27B****,** a femur having five optimized resection cuts for matching to an optimized five-bone-cut implant component; in **FIG. 27C,** a femur having five, not flexed resection cuts for matching to an optimized five-bone-cut implant component; and in **FIG. 27D,** a femur having five, flexed resection cuts for matching to an optimized five-bone-cut, flexed implant component. As shown in each of these figures, the designed bone cuts save substantial bone as compared to those required by a traditional implant component.

In summary, the component designs described in this example can save patient bone stock as compared to a traditional implant component and thereby allow the implant to be pre-primary. Alternatively or in addition, the implant components may include cut planes (i.e., of resection cuts and bone cuts) that are optimized based on patient-specific data to meet one or more user-defined parameters, as described above. For example, cut planes can be symmetric or asymmetric, parallel or non-parallel, aligned perpendicular to the sagittal plane or not perpendicular, varied from medial to lateral condyle, and/or can include other orientations. The cut plane designs may include a "flexed" (i.e., rotated or offset relative to the biomechanical or anatomical axes) orientation. Moreover, the design of attachment pegs may also be flexed relative to the biomechanical or anatomical axes.

## Claims

1. A method of designing an implant for repairing a joint of a patient, comprising:
obtaining, in a computer system, two-dimensional or three-dimensional representation of the joint of the patient using electronic image data of the joint of the patient and
optionally one or more joints associated with the joint of the patient;
obtaining a patient-specific biomotion model using the two-dimensional or three-dimensional representation;
designing an implant having a shape based on the two-dimensional or three-dimensional representation;
optimizing the shape using the patient-specific biomotion model; and
designing an optimized implant having the optimized shape wherein the shape includes at least one of a bone-facing or a joint-facing surface of the implant.

2. The method of claim 1, wherein the joint of the patient is a knee joint of the patient.

3. The method of claim 2, wherein the one or more joints associated with the joint of the patient is an ankle or hip joint associated with the knee joint of the patient.

4. The method of claim 2, wherein the shape of the implant is a shape of the implant in a sagittal plane.

5. The method of claim 4, wherein the shape of the implant is further optimized to include a standard, non-patient specific curve in a coronal plane.

6. The method of claim 1, wherein the implant is a femoral implant configured for the knee joint of the patient.

7. The method of claim 6, wherein the femoral implant is configured for replacing a single condyle of the knee joint of the patient.

8. The method of claim 1, wherein the patient-specific biomotion model is obtained by modifying a standard biomotion model from a reference database using patient-specific parameters.

9. The method of claim 8, wherein the patient-specific parameters include one or more parameters selected from the patient's age, gender, weight, height, body mass index, and race, a desired limb alignment or deformity correction, and the patient's electronic imaging data.

## Patentansprüche

1. Ein Verfahren zum Entwerfen eines Implantats zur Reparatur eines Gelenks eines Patienten umfassend:
Erhalten einer zweidimensionalen oder dreidimensionalen Darstellung des Gelenks des Patienten in einem Computersystem unter Verwendung von elektronischen Bilddaten des Gelenks des Patienten und gegebenenfalls eines oder mehrerer Gelenke, die mit dem Gelenk des Patienten assoziiert sind;
Erhalten eines patientenspezifischen biologischen Bewegungs-Modells unter Verwendung der zweidimensionalen oder dreidimensionalen Darstellung;
Entwerfen eines Implantats mit einer Form basierend auf der zweidimensionalen oder dreidimensionalen Darstellung;
Optimieren der Form unter Verwendung des patientenspezifischen biologischen Bewegung-Modells; und
Entwerfen eines optimierten Implantats mit optimierter Form, wobei die Form zumindest eine von einer knochenzugewandten oder einer gelenkzugewandten Oberfläche des Implantats beinhaltet.

2. Das Verfahren gemäß Anspruch 1, wobei das Gelenk des Patienten ein Kniegelenk des Patienten ist.

3. Das Verfahren gemäß Anspruch 2, wobei das eine oder die mehreren Gelenke, die mit dem Gelenk des Patienten assoziiert sind, ein Knöchel-oder Hüftgelenk ist, das mit dem Kniegelenk des Patienten assoziiert ist.

4. Das Verfahren gemäß Anspruch 2, wobei die Form des Implantats eine Form des Implantats in einer Sagittalebene ist.

5. Das Verfahren gemäß Anspruch 4, wobei die Form des Implantats weiter optimiert wird, um eine Standard-, nicht-patientenspezifische Krümmung in einer koronalen Ebene einzuschließen.

6. Das Verfahren gemäß Anspruch 1, wobei das Implantat ein Femurimplantat ist, das für das Kniegelenk des Patienten konfiguriert ist.

7. Das Verfahren gemäß Anspruch 6, wobei das Femurimplantat konfiguriert ist, um eine einzelne Kondyle des Kniegelenks des Patienten zu ersetzen.

8. Das Verfahren gemäß Anspruch 1, wobei das patientenspezifische biologische Bewegung-Modell durch Modifizieren eines standard-biologischen Bewegungsmodells aus einer Referenzdatenbank unter Verwendung patientenspezifischer Parameter erhalten wird.

9. Das Verfahren gemäß Anspruch 8, wobei die patientenspezifischen Parameter einen oder mehrere Parameter umfassen, die aus dem Alter, dem Geschlecht, dem Gewicht, der Größe, dem Body-Maß-Index und der Rasse und einer gewünschten Gliedmaßenausrichtung oder einer Deformitätsskorrektur und den elektronischen Bilddaten des Patienten ausgewählt sind.

## Revendications

1. Procédé de conception d'un implant pour réparer une articulation d'un patient, comprenant :
l'obtention, dans un système informatique, d'une représentation bidimensionnelle ou tridimensionnelle de l'articulation du patient à l'aide de données d'images électroniques de l'articulation du patient et optionnellement d'une ou de plusieurs articulations associées à l'articulation du patient ;
l'obtention d'un modèle de mouvement biologique spécifique au patient en utilisant la représentation bidimensionnelle ou tridimensionnelle ;
la conception d'un implant présentant une forme basée sur la représentation bidimensionnelle ou tridimensionnelle ;
l'optimisation de la forme à l'aide du modèle de mouvement biologique spécifique au patient ; et
la conception de l'implant optimisé présentant la forme optimisée
dans lequel la forme inclut au moins l'une d'une surface de l'implant faisant face à l'os ou faisant face à l'articulation.

2. Procédé selon la revendication 1, dans lequel l'articulation du patient est une articulation du genou du patient.

3. Procédé selon la revendication 2, dans lequel la ou les articulations associées à l'articulation du patient sont une cheville ou une articulation de la hanche associée à l'articulation du genou du patient.

4. Procédé selon la revendication 2, dans lequel la forme de l'implant est une forme de l'implant dans un plan sagittal.

5. Procédé selon la revendication 4, dans lequel la forme de l'implant est en outre optimisée pour inclure une courbe standard, non spécifique au patient dans un plan coronal.

6. Procédé selon la revendication 1, dans lequel l'implant est un implant fémoral configuré pour l'articulation du genou du patient.

7. Procédé selon la revendication 6, dans lequel l'implant fémoral est configuré pour remplacer un seul condyle de l'articulation du genou du patient.

8. Procédé selon la revendication 1, dans lequel le modèle de mouvement biologique spécifique au patient est obtenu en modifiant un modèle de mouvement biologique standard à partir d'une base de données de référence en utilisant des paramètres spécifiques au patient.

9. Procédé selon la revendication 8, dans lequel les paramètres spécifiques au patient incluent un ou plusieurs paramètres sélectionnés parmi l'âge, le sexe, le poids, la taille, l'indice de masse corporelle, et l'origine ethnique du patient, un alignement souhaité des membres ou une correction de malformation, et les données d'images électronique du patient.
